# EUROPEAN PATENT APPLICATION

(11) **EP 4 378 932 A1**
(43) Date of publication of application: **05.06.2024**
(21) Application number: 22210697.3
(22) Date of filing: 30.11.2022
(51) Int. Cl.: C07D 277/48, C07D 403/06, C07D 403/10, C07D 409/06, C07D 417/06, C07D 471/04, C07D 487/04

(54) **NOVEL CD93 INHIBITORS**

(71) Applicant: Universität Bern, 3012 Bern (CH)
(72) Inventor: RIEDL, Rainer, 8832 Wilen b. Wollerau (CH); BRAND, Michael, 5034 Suhr (CH); BOMMELI, Elias, 8105 Regensdorf (CH); OCHSENBEIN, Adrian, 3032 Hinterkappelen (CH); RIETHER, Carsten, 3012 Bern (CH)
(74) Representative: Schulz Junghans Patentanwälte PartGmbB

(57) **Abstract**

The present invention relates to a compound of formula (A)

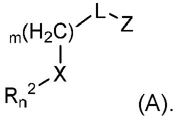

## Description

### Field

The present invention relates to CD93 inhibitors for use in the treatment of cancer.

### Background

Haematological malignancies such as leukemias, lymphomas and myelomas account for 9.5% of new cancer diagnoses in the United States. Treatment ranges from "watchful waiting" and symptomatic treatment to chemotherapy, radiotherapy, immunotherapy and even bone marrow transplantation. As the five-year survival rate e.g. for leukemia is nevertheless below 60 % in the United States, there is still a need for novel drugs and treatment approaches.

The introduction of BCR/ABL-specific tyrosine kinase inhibitors (TKIs) more than two decades ago revolutionized chronic myelogenous leukemia (CML) therapy. The majority of CML patients treated with TKIs obtain durable cytogenetic and molecular responses. However, only a subgroup of these patients can successfully discontinue TKI therapy and maintain a treatment-free remission. TKI-resistant leukemia stem cells (LSCs) persist in the majority of patients at low levels over a prolonged period. These quiescent, self-renewing LSCs in the bone marrow (BM) are the major cause of relapse after drug discontinuation (Holyoake et al, 2017). The selective elimination of LSCs requires the definition of unique signaling pathways that promote self-renewal of LSCs but not of normal hematopoietic stem cells (HSCs). Based on the expression of CD93 on LSCs in chronic myeloid leukemia (Kinstrie et al, 2015, Riether et al, 2021) and acute myeloid leukemia (Iwasaki et al, 2015), the present invention aims to provide inhibitors of the cell surface receptor CD93, which regulates the self-renewal of human and murine CML LSCs and contributes to disease development and progression.

Based on the above-mentioned state of the art, the objective of the present invention is to provide compounds for use in the treatment of cancer. This objective is attained by the subject-matter of the independent claims of the present specification, with further advantageous embodiments described in the dependent claims, examples, figures and general description of this specification.

### Summary of the Invention

A first aspect of the invention relates to a compound of formula (A) 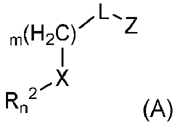 wherein
L is selected from -CO, -SO₂ or -(CH₂)_{q},
Z is selected from 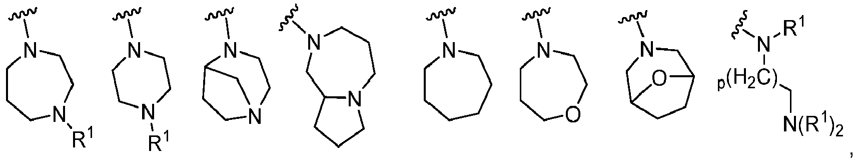
R¹ is selected from
   - -C₃-C₆ cycloalkyl,
   - -H,
   - -C₁-C₆- alkyl,
   - haloalkanes, particularly fluorinated alkanes,
   - a heterocycle, wherein the heterocycle comprises 5 or 6 atoms and up to 2 heteroatoms,
   - -COCH₃,
X is selected from
   - a heteroaryl,
   - phenyl,
   - SP-phenyl or SP-heteroaryl, wherein SP is an alkyne or alkene,
Rₙ² is selected from
   - -C₁-C₄ alkyl, particularly methyl,
   - an aromatic moiety (Ar), wherein Ar is an substituted or unsubstituted aryl or substituted or unsubstituted heteroaryl
   - the group of halogens, particularly Br
   - a heterocycle, wherein the heterocycle comprises 5 or 6 atoms and up to 3 heteroatoms,
   - -NMe₂, -OMe, -SO₂Me, -CONHMe,
m is 0 or 1, particularly 0,
n is 0, 1 or 2, particularly 0 or 1,
p is 1 or 2,
q is 0, 1 or 2, particularly 0 or 1.

A second aspect of the invention relates to a compound according to the first aspect of the invention for use as a medicament.

A third aspect of the invention relates to a compound according to the first aspect of the invention for use in the treatment of a disease, wherein the disease is cancer.

### Terms and definitions

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising", "having", "containing", and "including", and other similar forms, and grammatical equivalents thereof, as used herein, are intended to be equivalent in meaning and to be open-ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictates otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a", "or" and "the" include plural referents unless the context clearly dictates otherwise.

"And/or" where used herein is to be taken as specific recitation of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

Unless defined otherwise, all technical and scientific terms used herein have the same meaning as commonly understood by one of ordinary skill in the art (e.g., in cell culture, molecular genetics, nucleic acid chemistry, hybridization techniques and biochemistry, organic synthesis). Standard techniques are used for molecular, genetic, and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

Any patent document cited herein shall be deemed incorporated by reference herein in its entirety.

The term *gene* refers to a polynucleotide containing at least one open reading frame (ORF) that is capable of encoding a particular polypeptide or protein after being transcribed and translated. A polynucleotide sequence can be used to identify larger fragments or full-length coding sequences of the gene with which they are associated. Methods of isolating larger fragment sequences are known to those of skill in the art.

In the context of the present invention, the term "CD93" or "Cd93" relates to the receptor encoded by the murine gene ENSMUSG00000027435; *mCd93,* or the human CD93 gene (HGNC ID: 15855; Entrez Gene ID: 22918; Ensembl: ENSG00000125810; UniProtKB: Q9NPY3). Furthermore, the term "CD93" or "Cd93" includes homologs or variants thereof.

In the context of the present invention, the term "CD93 intracellular domain" or "Cd93^{intra"} lacks the sequence encoding for the extracellular domain of Cd93. The CD93 intracellular domain consists of the signal peptide (1-22aa), transmembrane (574-598aa) and cytoplasmic (599-644aa) domains of the Cd93 open reading frame (ORF) (total length of 282bp).

The term *C₁-C₄ alkyl* in the context of the present specification relates to a saturated linear or branched hydrocarbon having 1, 2, 3 or 4 carbon atoms, wherein in certain embodiments one carbon-carbon bond may be unsaturated and one CH₂ moiety may be exchanged for oxygen (ether bridge) or nitrogen (NH, or NR with R being methyl, ethyl, or propyl; amino bridge). Non-limiting examples for a C₁-C₄ alkyl are methyl, ethyl, propyl, prop-2-enyl, n-butyl, 2-methylpropyl, *tert*-butyl, but-3-enyl, prop-2-inyl and but-3-inyl. In certain embodiments, a C₁-C₄ alkyl is a methyl, ethyl, propyl or butyl moiety.

A *C₁-C₆ alkyl* in the context of the present specification relates to a saturated linear or branched hydrocarbon having 1, 2, 3, 4, 5 or 6 carbon atoms, wherein one carbon-carbon bond may be unsaturated and/or one CH₂ moiety may be exchanged for oxygen (ether bridge) or nitrogen (NH, or NR with R being methyl, ethyl, or propyl; amino bridge). Non-limiting examples for a C₁-C₆ alkyl include the examples given for C₁-C₄ alkyl above, and additionally 3-methylbut-2-enyl, 2-methylbut-3-enyl, 3-methylbut-3-enyl, n-pentyl, 2-methylbutyl, 3-methylbutyl, 1,1-dimethylpropyl, 1,2-dimethylpropyl, 1,2-dimethylpropyl, pent-4-inyl, 3-methyl-2-pentyl, and 4-methyl-2-pentyl. In certain embodiments, a C₅ alkyl is a pentyl or cyclopentyl moiety and a C₆ alkyl is a hexyl or cyclohexyl moiety.

A *C₃-C₆ cycloalkyl* in the context of the present specification relates to a saturated cyclic hydrocarbon having 3, 4, 5 or 6 carbon atoms. Examples for a C₃-C₆ cycloalkyl are cyclopropyl, cyclobutyl, cyclopentyl, and cyclohexyl.

The term *heterocycle* in the context of the present specification relates to a saturated cyclic hydrocarbon comprising 5, 6, 7, 8, 9 or 10 atoms of which up to 3 atoms are heteroatoms. Non-limiting examples for heteroatoms are N, S and O.

The term *aryl* in the context of the present specification relates to a cyclic aromatic C₅-C₁₀ hydrocarbon that may comprise a heteroatom (e.g. N, O, S). Examples of aryl include, without being restricted to, phenyl and naphthyl, and any heteroaryl. A *heteroaryl* is an aryl that comprises one or several nitrogen, oxygen and/or sulphur atoms. Examples for heteroaryl include, without being restricted to, pyrrole, thiophene, furan, imidazole, pyrazole, thiazole, oxazole, pyridine, pyrimidine, thiazin, quinoline, benzofuran and indole. An aryl or a heteroaryl in the context of the specification additionally may be substituted by one or more C₁-C₆ alkyl groups as defined herein, -O-C₁-C₆-alkyl moieties, -NHSO₂C₁-C₆ alkyl moieties, a halogen, -NO₂, -CN, -NH-C₁-C₆-alkyl, -N-(C₁-C₆-alkyl)₂, -SO₂-C₁-C₆-alkyl, -OCF₃, -OCHF₂, - OCH₂F, -CF₃, -CHF₂, -CH₂F or the like.

As used herein, the term *pharmaceutical composition* refers to a compound of the invention, or a pharmaceutically acceptable salt thereof, together with at least one pharmaceutically acceptable carrier. In certain embodiments, the pharmaceutical composition according to the invention is provided in a form suitable for topical, parenteral or injectable administration.

The term *cancer* as used in the context of the present specification relates to malignant neoplastic disease; the terms "cancer" and "malignant neoplastic disease" are used synonymously herein. They specifically include carcinoma (epithelial derived cancer), sarcoma (connective tissue derived cancer), lymphoma and leukemia, germ-cell derived tumours and blastomas. Particular alternatives of any of the aspects and embodiments disclosed herein are directed at the use of the compounds and compositions of the invention in treatment of solid tumours. Other alternatives of any of the aspects and embodiments disclosed herein are directed at the use of the combinations of the invention in treatment of liquid cancers such as myelogenous or granulocytic leukemia, particularly AML, lymphatic, lymphocytic, or lymphoblastic leukemia and lymphoma, polycythemia vera or erythremia.

The term *inhibitor* in the context of the present specification relates to any pharmaceutically acceptable agent or compound that may be used to interact with and specifically interfere with the biological activity of its designated target.

As used herein, the term *treating* or *treatment* of any disease or disorder (e.g. cancer) refers in one embodiment, to ameliorating the disease or disorder (e.g. slowing or arresting or reducing the development of the disease or at least one of the clinical symptoms thereof). In another embodiment "treating" or "treatment" refers to alleviating or ameliorating at least one physical parameter including those which may not be discernible by the patient. In yet another embodiment, "treating" or "treatment" refers to modulating the disease or disorder, either physically, (e.g., stabilization of a discernible symptom), physiologically, (e.g., stabilization of a physical parameter), or both. Methods for assessing treatment and/or prevention of disease are generally known in the art, unless specifically described hereinbelow.

### Detailed Description of the Invention

A first aspect of the invention relates to a compound of formula (A) 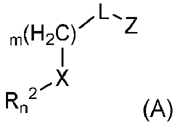 wherein
L is selected from -CO, -SO₂ or -(CH₂)_{q},
Z is selected from 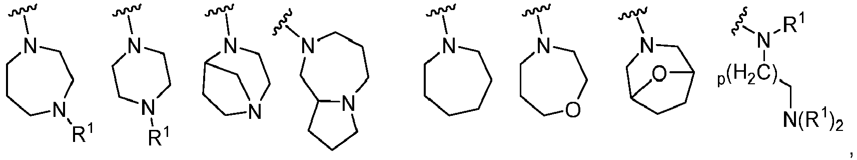
R¹ is selected from
   - -C₃-C₆ cycloalkyl,
   - -H,
   - -C₁-C₆- alkyl,
   - haloalkanes, particularly fluorinated alkanes,
   - a heterocycle, wherein the heterocycle comprises 5 or 6 atoms and up to 2 heteroatoms,
   - -COCH₃,
X is selected from
   - a heteroaryl,
   - phenyl,
   - SP-phenyl or SP-heteroaryl, wherein SP is an alkyne or alkene,
Rₙ² is selected from
   - -C₁-C₄ alkyl, particularly methyl,
   - an aromatic moiety (Ar), wherein Ar is an substituted or unsubstituted aryl or substituted or unsubstituted heteroaryl
   - the group of halogens, particularly Br
   - a heterocycle, wherein the heterocycle comprises 5 or 6 atoms and up to 3 heteroatoms,
   - -NMe₂, -OMe, -SO₂Me, -CONHMe, -NH-Phenyl
      m is 0 or 1, particularly 0,
      n is 0, 1 or 2, particularly 0 or 1,
      p is 1 or 2,
      q is 0, 1 or 2, particularly 0 or 1.

In certain embodiments, the compound is a compound of formula (I), (II), or (III) , 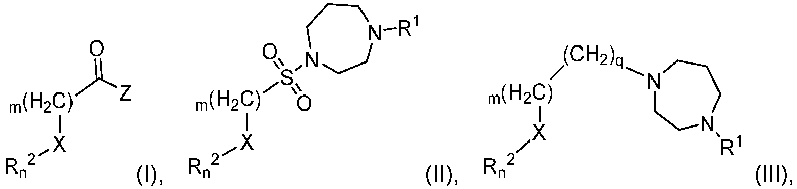 wherein
Z is selected from 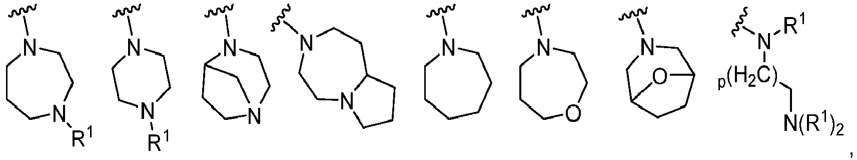
R¹ is selected from
   - -C₃-C₆ cycloalkyl,
   - -H,
   - -C₁-C₆- alkyl,
   - haloalkanes, particularly fluorinated alkanes,
   - a heterocycle, wherein the heterocycle comprises 5 or 6 atoms and up to 3 heteroatoms,
   - -COCH₃,
X is selected from
   - a heteroaryl,
   - phenyl,
   - SP-phenyl or SP-heteroaryl, wherein SP is an alkyne or alkene,
Rₙ² is selected from
   - -C₁-C₄ alkyl, particularly methyl,
   - an aromatic moiety (Ar), wherein Ar is an substituted or unsubstituted aryl or substituted or unsubstituted heteroaryl
   - the group of halogens, particularly Br
   - a heterocycle, wherein the heterocycle comprises 5 or 6 atoms and up to 3 heteroatoms, particularly up to 2 heteroatoms,
   - -NMe₂, -OMe, -SO₂Me, -CONHMe, -NH-Phenyl,
      m is 0 or 1, particularly 0,
      n is 0, 1 or 2, particularly 0 or 1,
      p is 1 or 2,
      q is 0, 1 or 2, particularly 0 or 1.

In certain embodiments, the heterocycle comprises up to 2 heteroatoms.

In certain embodiments R¹ is selected from
- -C₃-C₆ cycloalkyl, particularly cyclopropyl,
- -H,
- -C₁-C₆- alkyl.

In certain embodiments R¹ is selected from
- -C₃-C₆ cycloalkyl, particularly cyclopropyl,
- -C₁-C₆- alkyl.

In certain embodiments R¹ is selected from
- -C₃-C₆ cycloalkyl.

In certain embodiments R¹ is cyclopropyl.

In certain embodiments, X is a phenyl or heteroaryl and wherein the heteroaryl comprises 5 to 10 atoms.

In certain embodiments, the heteroaryl comprises 5 to 9 atoms.

In certain embodiments, the heteroaryl comprises 5 atoms.

In certain embodiments, the heteroaryl of R² and the heteroaryl of X independently comprise up to 3 heteroatoms.

In certain embodiments, the heteroaryl of R² and the heteroaryl of X independently comprise up to 2 heteroatoms.

In certain embodiments, the heteroaryl of R² and the heteroaryl of X independently comprise 2 heteroatoms.

In certain embodiments, X-Rₙ² is selected from 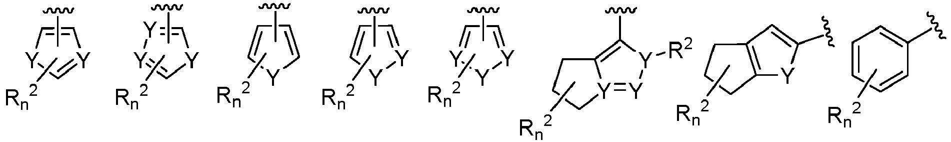 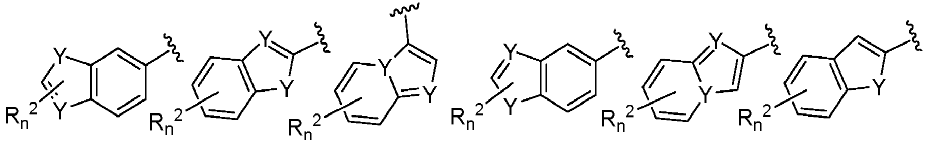 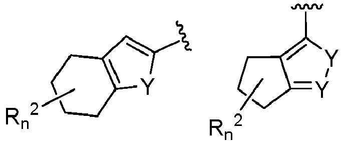 wherein
Y is N, O or S,
R² is selected from
   - -C₁-C₄ alkyl, particularly methyl,
   - an aromatic moiety (Ar), wherein Ar is an substituted or unsubstituted aryl or substituted or unsubstituted heteroaryl
   - the group of halogens, particularly Br
   - a heterocycle, wherein the heterocycle comprises 5 or 6 atoms and up to 3 heteroatoms, particularly up to 2 heteroatoms,
   - -NMe₂, -OMe, -SO₂Me, -CONHMe,
n is 0, 1 or 2, particularly 0 or 1.

In certain embodiments, X-Rₙ² is selected from 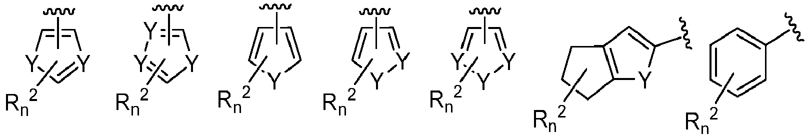 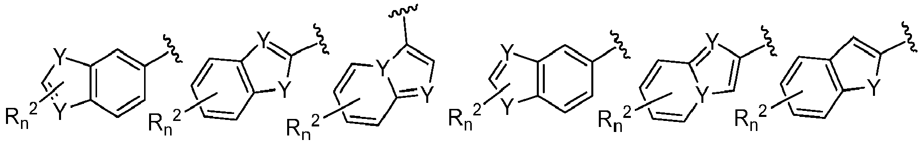 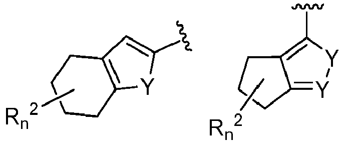 wherein
Y is N, O or S,
R² is selected from
   - -C₁-C₄ alkyl, particularly methyl,
   - an aromatic moiety (Ar), wherein Ar is an substituted or unsubstituted aryl or substituted or unsubstituted heteroaryl
   - the group of halogens, particularly Br
   - a heterocycle, wherein the heterocycle comprises 5 or 6 atoms and up to 3 heteroatoms, particularly up to 2 heteroatoms,
   - -NMe₂, -OMe, -SO₂Me, -CONHMe,
n is 0, 1 or 2, particularly 0 or 1.

In certain embodiments, X-Rₙ² is selected from 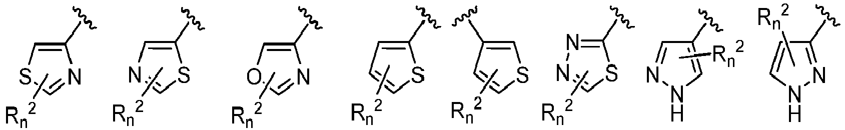 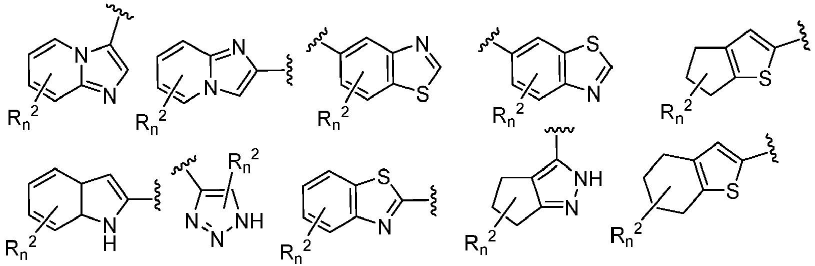 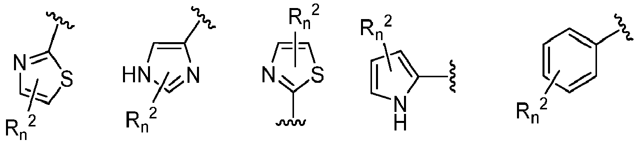 wherein
R² is selected from
   - -C₁-C₄ alkyl, particularly methyl,
   - an aromatic moiety (Ar), wherein Ar is an substituted or unsubstituted aryl or substituted or unsubstituted heteroaryl
   - the group of halogens, particularly Br
   - a heterocycle, wherein the heterocycle comprises 5 or 6 atoms and up to 3 heteroatoms, particularly up to 2 heteroatoms,
   - -NMe₂, -OMe, -SO₂Me, -CONHMe,
n is 0 or 1.

In certain embodiments, X-R² is selected from 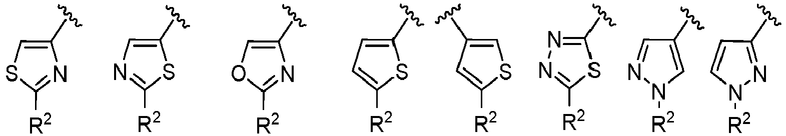 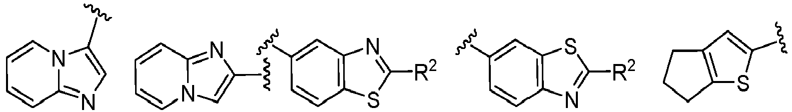 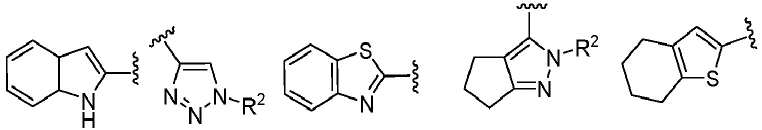 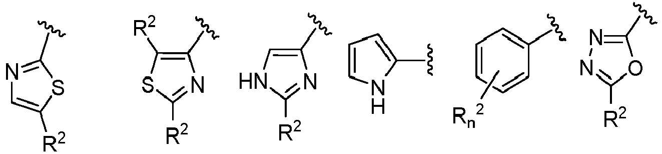 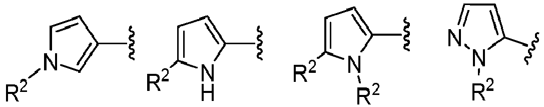 wherein
R² is selected from
- C₁-C₄ alkyl, particularly methyl,
- an aromatic moiety (Ar), wherein Ar is an substituted or unsubstituted aryl or substituted or unsubstituted heteroaryl
- the group of halogens, particularly Br
- a heterocycle, wherein the heterocycle comprises 5 or 6 atoms and up to 3 heteroatoms, particularly up to 2 heteroatoms,
- -NMe₂, -OMe, -SO₂Me, -CONHMe,
n is 0 or 1.

In certain embodiments, X-R² is selected from 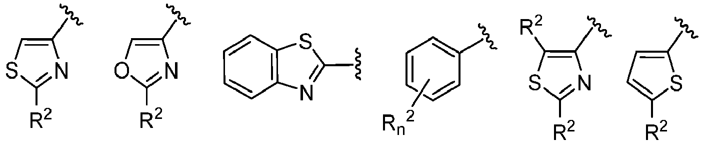 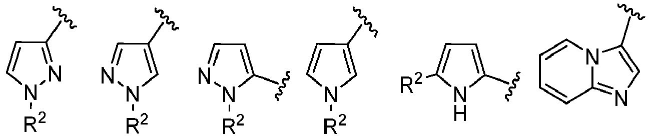 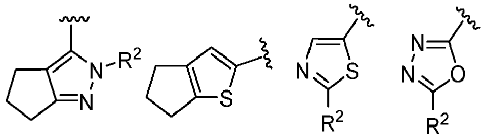 wherein
R² is selected from
- -C₁-C₄ alkyl, particularly methyl,
- an aromatic moiety (Ar), wherein Ar is an substituted or unsubstituted aryl or substituted or unsubstituted heteroaryl
- the group of halogens, particularly Br
- a heterocycle, wherein the heterocycle comprises 5 or 6 atoms and up to 3 heteroatoms, particularly up to 2 heteroatoms,
- -NMe₂, -OMe, -SO₂Me, -CONHMe,
n is 0 or 1.

In certain embodiments, R² is selected from
- -C₁-C₄ alkyl, particularly methyl,
- Ar, wherein Ar is a substituted or unsubstituted aryl or a substituted or unsubstituted heteroaryl
- the group of halogens, particularly Br
- a heterocycle, wherein the heterocycle comprises 5 or 6 atoms and up to 2 heteroatoms, particularly piperidine,
- -NMe₂, -OMe, -SO₂Me, -CONHMe, -NH-Phenyl.

In certain embodiments, R² is selected from
- -C₁-C₄ alkyl, particularly methyl,
- Ar, wherein Ar is a substituted or unsubstituted aryl or a substituted or unsubstituted heteroaryl
- a heterocycle, wherein the heterocycle comprises 5 or 6 atoms and up to 2 heteroatoms, particularly piperidine
- the group of halogens, particularly Br.

In certain embodiments, Ar comprises 5 to 10 atoms.

In certain embodiments, Ar comprises 5 to 9 atoms.

In certain embodiments, the substituted aryl or the substituted heteroaryl is substituted with Rₛ³, wherein s is 0, 1 or 2, particularly 0 or 1.

In certain embodiments, R³ is selected from
- -C₁-C₄ alkyl, particularly methyl,
- the group of halogens, particularly F or Cl,
- -CN, -NO₂, -OCF₃, -NMe₂, -CONHMe, -OMe, -SO₂Me, -NHSO₂Me, - CF₃, -Me.

In a certain embodiments, the compound is a compound of formula (IV), (IVa), (V), or (VII), particularly of (IV), (V) or (VII) 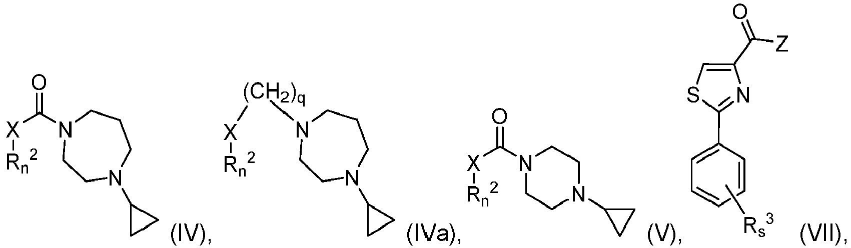 wherein
q is 0 or 1, particularly 0,
X is selected from 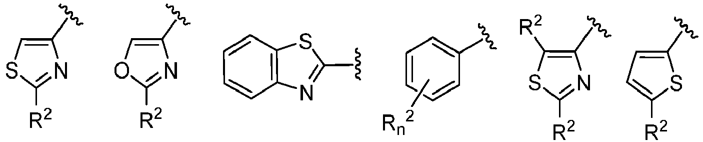 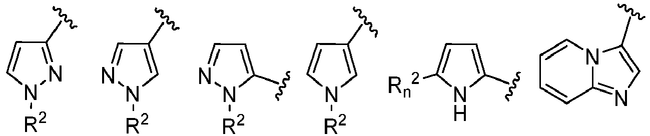 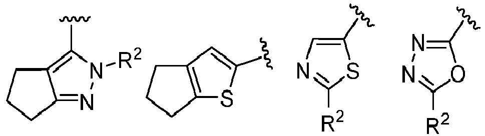
R² is selected from
   - -C₁-C₄ alkyl, particularly methyl,
   - Ar, wherein Ar is a substituted or unsubstituted aryl or a substituted or unsubstituted heteroaryl,
   - a heterocycle, wherein the heterocycle comprises 5 or 6 atoms and up to 2 heteroatoms, particularly piperidine,
   - the group of halogens, particularly Br,
R³ is selected from
   - -C₁-C₄ alkyl, particularly methyl,
   - the group of halogens, particularly F or Cl,
   - -CN, -NO₂, -OCF₃, -NMe₂, -CONHMe, -OMe, -SO₂Me, -NHSO₂Me, -CF₃, -Me,
Z is selected from 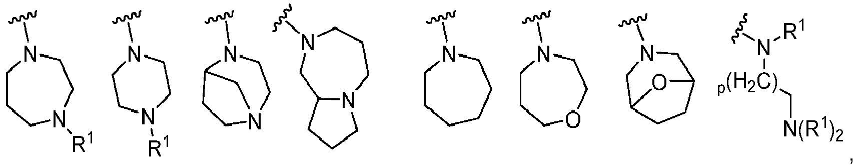
R¹ is selected from
   - methyl and cyclopropyl,
n is 0 or 1,
p is 1 or 2,
s is 0 or 1.

In a certain embodiments, the compound is a compound of formula (IV), (IVa), (V), or (VIII), particularly of (IV), (V) or (VII) 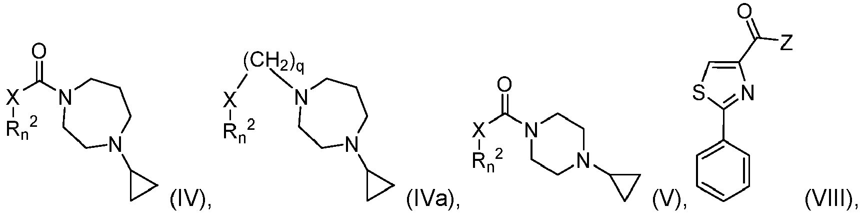 wherein
q is 0 or 1, particularly 0,
X is selected from 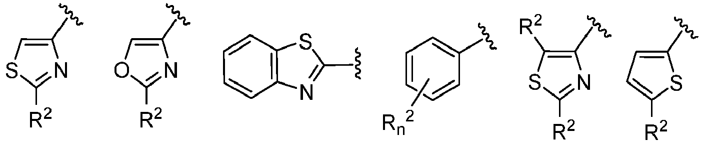 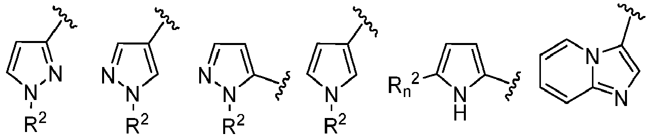 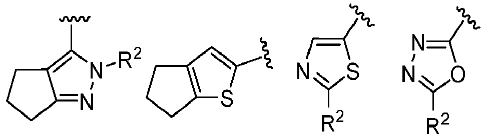
R² is selected from
   - -C₁-C₄ alkyl, particularly methyl,
   - Ar, wherein Ar is a substituted or unsubstituted aryl or a substituted or unsubstituted heteroaryl,
   - a heterocycle, wherein the heterocycle comprises 5 or 6 atoms and up to 2 heteroatoms, particularly piperidine
   - the group of halogens, particularly Br,
R³ is selected from
   - -C₁-C₄ alkyl, particularly methyl,
   - the group of halogens, particularly F or Cl,
   - -CN, -NO₂, -OCF₃, -NMe₂, -CONHMe, -OMe, -SO₂Me, -NHSO₂Me, -CF₃, -Me,
Z is selected from 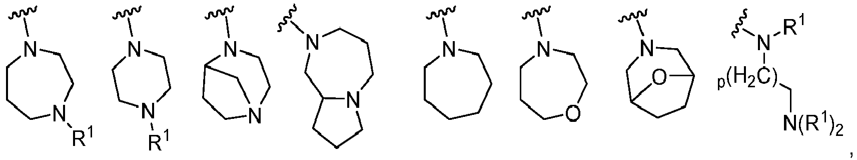
R¹ is selected from
   - methyl and cyclopropyl,
n is 0 or 1,
p is 1 or 2,
s is 0 or 1.

In certain embodiments, the compound is a compound of formula (IV), (IVa), or (VIII) particularly of (IV) or (VII) 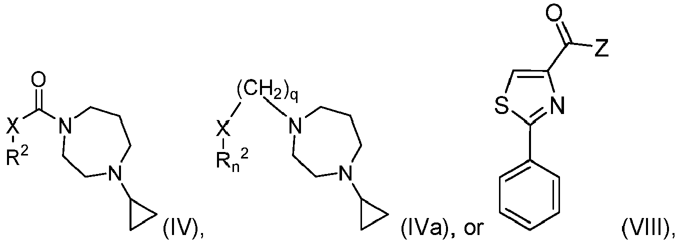 wherein
q is 0 or 1, particularly 0,
X is selected from 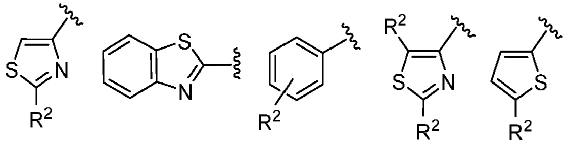 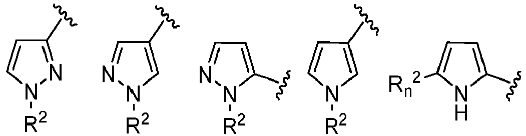 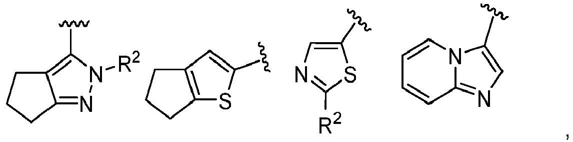
R² is selected from
   - Methyl, Br 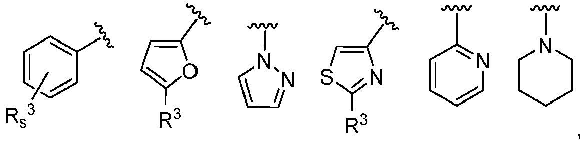
R³ is selected from
   - Methyl, -OMe, -NHSO₂Me, -Cl, -NO₂, -CN,
Z is selected from 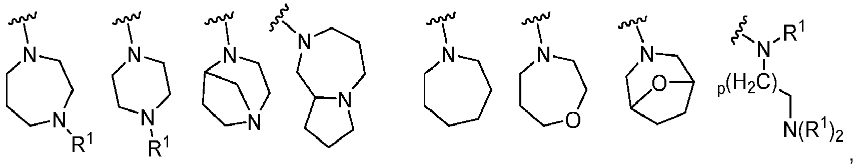
R¹ is selected from
   - methyl and cyclopropyl,
s is 0 or 1.

In certain embodiments, the compound is a compound of formula (VIII) 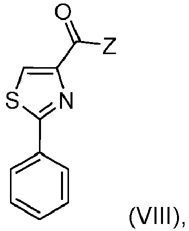 wherein
Z is selected from 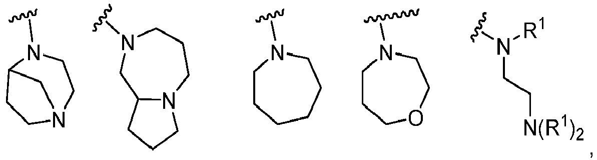
R¹ is selected from methyl and cyclopropyl.

In certain embodiments, the compound is a compound of formula (VIII) 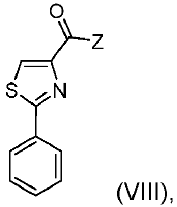 wherein
Z is selected from 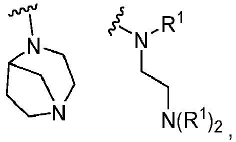
R¹ is selected from methyl and cyclopropyl.

In certain embodiments, the compound is a compound of formula 22745, 22747, 22817 or 22815 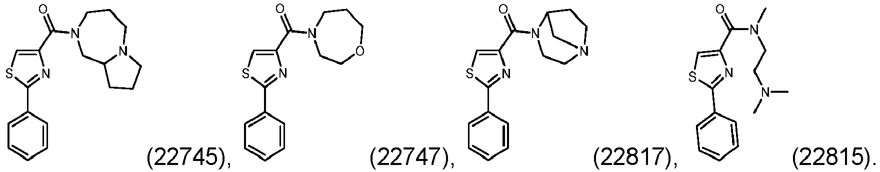 In certain embodiments, the compound is a compound of formula (22745) or (22815) 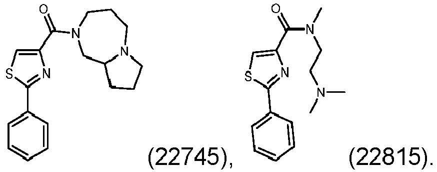 In certain embodiments, the compound is a compound of formula (IV) 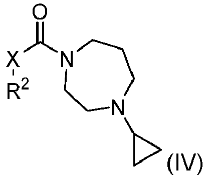 wherein
X is selected from 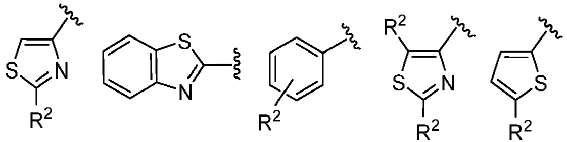 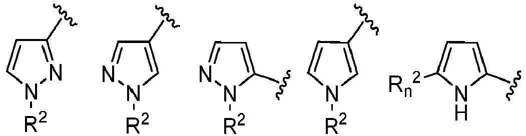 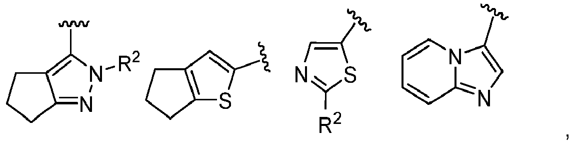
R² is selected from
   - Methyl, Br 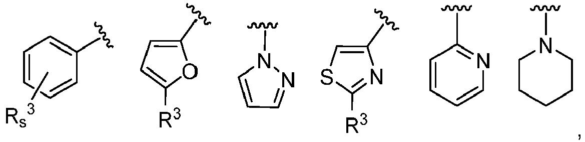
R³ is selected from
   - Methyl, -OMe, -NHSO₂Me, -Cl, -NO₂, -CN,
s is 0 or 1.

In certain embodiments, the compound is a compound of formula (IV) 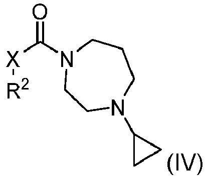 wherein
X is selected from 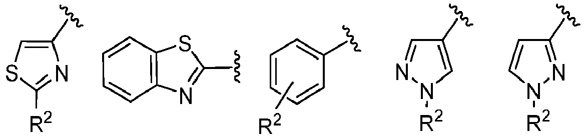 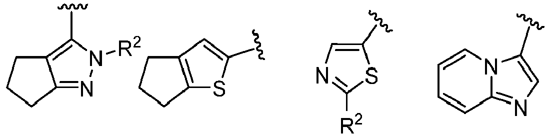
R² is selected from
   - methyl, Br,
   - 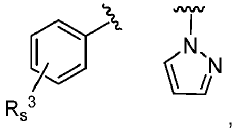
R³ is selected from
   - -OMe, -CN
s is 0 or 1.

In certain embodiments, the compound is a compound of formula (21560), (22741), (22742), (22749), (22394), (22393), (22396), (22850), (22635), (22608) or (22612)

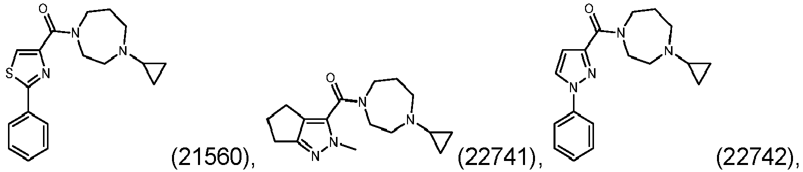

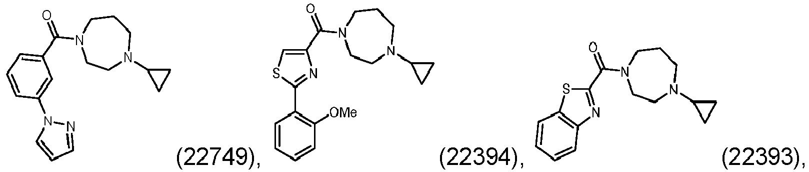

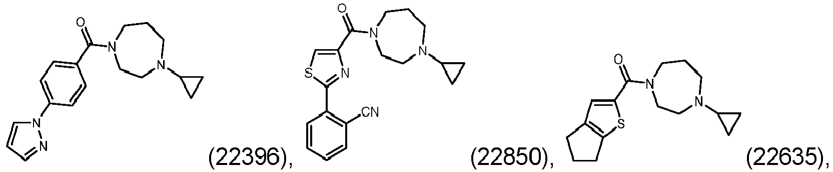

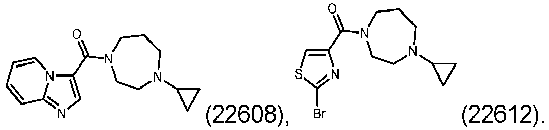

In certain embodiments, the compound is a compound of formula (21560), (22742), (22394), (22393), (22396) or (22635).

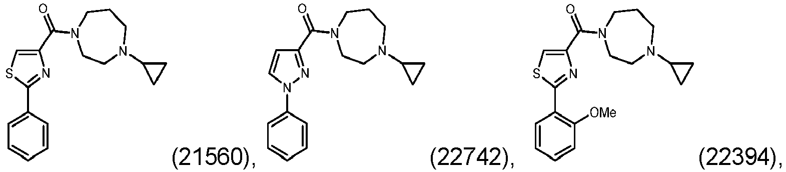

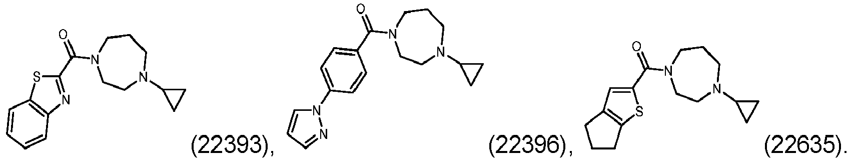

In certain embodiments, the compound is a compound of formula (21560), (22742), (22394), (22393) or (22396). 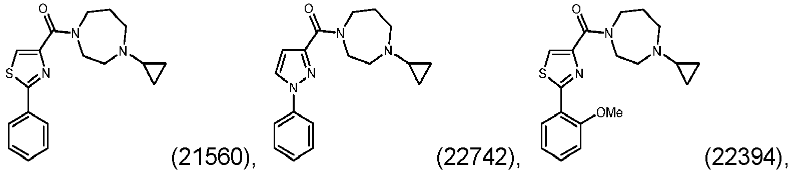 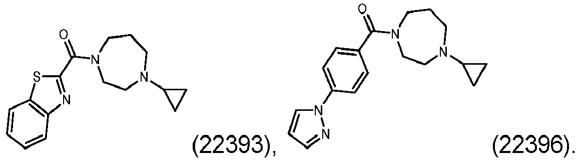 In certain embodiments, the compound is a compound of formula (IVa) 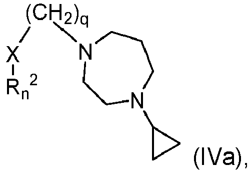 wherein
X is selected from 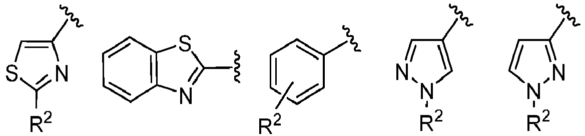 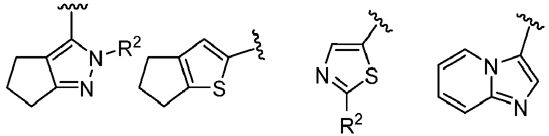
R² is selected from
   - methyl, Br,
   - 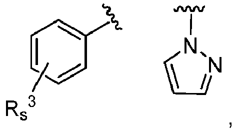
R³ is selected from
   - -OMe, -CN
s is 0 or 1.

In certain embodiments, the compound is a compound of formula (IX)

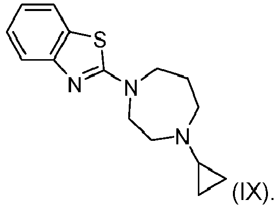

A second aspect of the invention relates to a compound according to the first aspect of the invention for use as a medicament.

A third aspect of the invention relates to a compound according to the first aspect of the invention for use in the treatment of a disease.

In certain embodiments, the disease is cancer.

In certain embodiments, the cancer is selected from leukemia, lymphoma or myeloma.

In certain embodiments, the cancer is selected from acute myeloid leukemia (AML), chronic myelogenous leukemia (CML) or multiple myeloma (MM).

In certain embodiments, the cancer is acute myeloid leukemia (AML).

The invention is further illustrated by the following examples and figures, from which further embodiments and advantages can be drawn. These examples are meant to illustrate the invention but not to limit its scope.

### Medical treatment

Similarly, within the scope of the present invention is a method or treating cancer in a patient in need thereof, comprising administering to the patient a compound according to the above description.

As used herein, the term *treating or treatment* of any disease or disorder (e.g. cancer) refers in one embodiment, to ameliorating the disease or disorder (e.g. slowing or arresting or reducing the development of the disease or at least one of the clinical symptoms thereof). In another embodiment "treating" or "treatment" refers to alleviating or ameliorating at least one physical parameter including those which may not be discernible by the patient. In yet another embodiment, "treating" or "treatment" refers to modulating the disease or disorder, either physically, (e.g., stabilization of a discernible symptom), physiologically, (e.g., stabilization of a physical parameter), or both. Methods for assessing treatment and/or prevention of disease are generally known in the art, unless specifically described hereinbelow.

### Pharmaceutical Compositions, Administration/Dosage Forms and Salts

According to one aspect of the compound according to the invention, the compound according to the invention is provided as a pharmaceutical composition, pharmaceutical administration form, or pharmaceutical dosage form, said pharmaceutical composition, pharmaceutical administration form, or pharmaceutical dosage form comprising at least one of the compounds of the present invention or a pharmaceutically acceptable salt thereof and at least one pharmaceutically acceptable carrier, diluent or excipient.

The skilled person is aware that any specifically mentioned drug compound mentioned herein may be present as a pharmaceutically acceptable salt of said drug. Pharmaceutically acceptable salts comprise the ionized drug and an oppositely charged counterion. Non-limiting examples of pharmaceutically acceptable anionic salt forms include acetate, benzoate, besylate, bitatrate, bromide, carbonate, chloride, citrate, edetate, edisylate, embonate, estolate, fumarate, gluceptate, gluconate, hydrobromide, hydrochloride, iodide, lactate, lactobionate, malate, maleate, mandelate, mesylate, methyl bromide, methyl sulfate, mucate, napsylate, nitrate, pamoate, phosphate, diphosphate, salicylate, disalicylate, stearate, succinate, sulfate, tartrate, tosylate, triethiodide and valerate. Non-limiting examples of pharmaceutically acceptable cationic salt forms include aluminium, benzathine, calcium, ethylene diamine, lysine, magnesium, meglumine, potassium, procaine, sodium, tromethamine and zinc.

In certain embodiments of the invention, the compound of the present invention is typically formulated into pharmaceutical dosage forms to provide an easily controllable dosage of the drug and to give the patient an elegant and easily handleable product.

Similarly, a dosage form for the prevention or treatment of cancer is provided, comprising a non-agonist ligand or antisense molecule according to any of the above aspects or embodiments of the invention.

The invention further encompasses a pharmaceutical composition comprising a compound of the present invention, or a pharmaceutically acceptable salt thereof, and a pharmaceutically acceptable carrier. In further embodiments, the composition comprises at least two pharmaceutically acceptable carriers, such as those described herein.

Certain embodiments of the invention relate to a dosage form for enteral administration, such as nasal, buccal, rectal, transdermal or oral administration, or as an inhalation form or suppository. In addition, the pharmaceutical compositions of the present invention can be made up in a solid form (including without limitation capsules, tablets, pills, granules, powders or suppositories), or in a liquid form (including without limitation solutions, suspensions or emulsions).

Certain embodiments of the invention relate to a dosage form for parenteral administration, such as subcutaneous, intravenous, intrahepatic or intramuscular injection forms. Optionally, a pharmaceutically acceptable carrier and/or excipient may be present.

Certain embodiments of the invention relate to a dosage form for topical administration. The skilled artisan is aware of a broad range of possible recipes for providing topical formulations, as exemplified by the content of Benson and Watkinson (Eds.), Topical and Transdermal Drug Delivery: Principles and Practice (1st Edition, Wiley 2011, ISBN-13: 978-0470450291); and Guy and Handcraft: Transdermal Drug Delivery Systems: Revised and Expanded (2nd Ed., CRC Press 2002, ISBN-13: 978-0824708610); Osborne and Amann (Eds.): Topical Drug Delivery Formulations (1st Ed. CRC Press 1989; ISBN-13: 978-0824781835). In embodiments of the invention relating to topical uses of the compounds of the invention, the pharmaceutical composition is formulated in a way that is suitable for topical administration such as aqueous solutions, suspensions, ointments, creams, gels or sprayable formulations, e.g., for delivery by aerosol or the like, comprising the active ingredient together with one or more of solubilizers, stabilizers, tonicity enhancing agents, buffers and preservatives that are known to those skilled in the art.

The dosage regimen for the compounds of the present invention will vary depending upon known factors, such as the pharmacodynamic characteristics of the particular agent and its mode and route of administration; the species, age, sex, health, medical condition, and weight of the recipient; the nature and extent of the symptoms; the kind of concurrent treatment; the frequency of treatment; the route of administration, the renal and hepatic function of the patient, and the effect desired. In certain embodiments, the compounds of the invention may be administered in a single daily dose, or the total daily dosage may be administered in divided doses of two, three, or four times daily.

The pharmaceutical compositions of the present invention can be subjected to conventional pharmaceutical operations such as sterilization and/or can contain conventional inert diluents, lubricating agents, or buffering agents, as well as adjuvants, such as preservatives, stabilizers, wetting agents, emulsifiers and buffers, etc. They may be produced by standard processes, for instance by conventional mixing, granulating, dissolving or lyophilizing processes. Many such procedures and methods for preparing pharmaceutical compositions are known in the art, see for example L. Lachman et al. The Theory and Practice of Industrial Pharmacy, 4th Ed, 2013 (ISBN 8123922892).

### Method of Manufacture and Method of Treatment according to the invention

The invention further encompasses, as an additional aspect, the use of a compound as identified herein, or its pharmaceutically acceptable salt, as specified in detail above, for use in a method of manufacture of a medicament for the treatment or prevention of cancer.

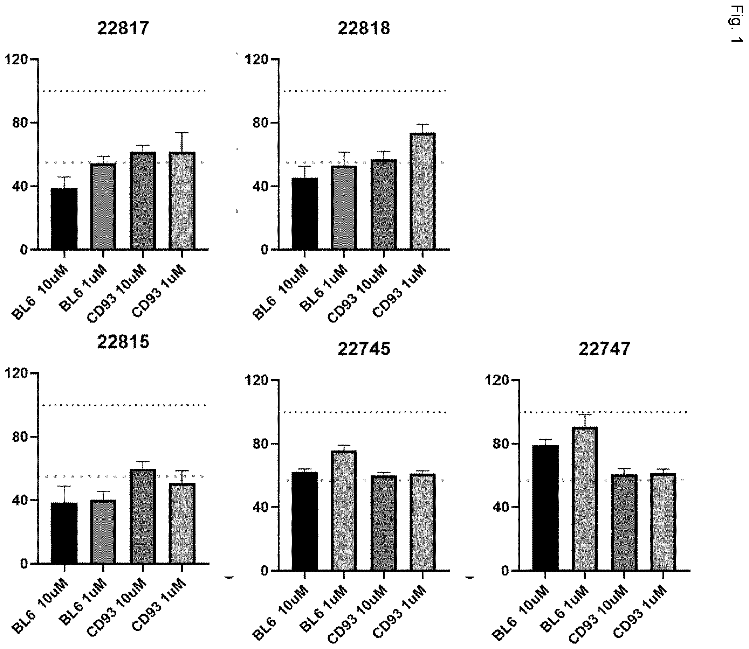

Similarly, the invention encompasses methods of treatment of a patient having been diagnosed with a disease associated with cancer. This method entails administering to the patient an effective amount of a compound as identified herein, or its pharmaceutically acceptable salt, as specified in detail herein. Description of the Figures
- Fig. 1: shows colony forming capacity from BL6 or CD93-/- GMPs (leukemia stem cells) derived from AML mice post compound dose. The colony formation (% of control) is shown on the Y-axis. The upper dotted line displays the BL6 control, the lower dotted line displays the CD93-/- control, normalized to the BL6 control. Compounds showing inhibition of CD93, show no or a very low difference in colony formation of CD93-/- and at the same time a low percentage of colony formation of BL6. Compounds 22817 and 22815 show good inhibition profiles of CD93.

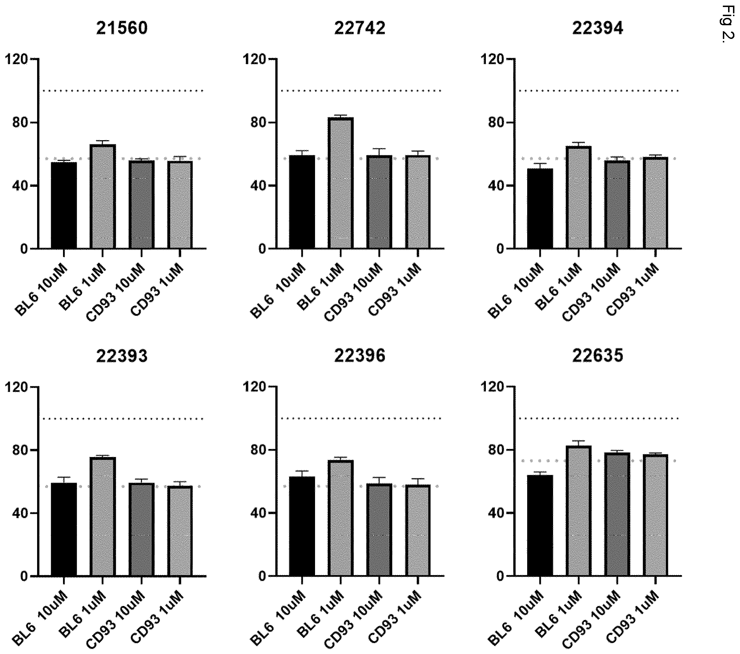

- Fig. 2: shows colony forming capacity from BL6 or CD93-/- GMPs derived from AML mice post compound dose. The colony formation (% of control) is shown on the Y-axis. The upper dotted line displays the BL6 control, the lower dotted line displays the CD93-/- control, normalized to the BL6 control. Compounds showing inhibition of CD93, show no or a very low difference in colony formation of CD93-/- and at the same time a low percentage of colony formation of BL6. Compounds 21560, 22742, 22394, 22393 and show good inhibition profiles of CD93.

### Examples

### Experimental details

All reagents and solvents were purchased from Sigma-Aldrich, Alfa Aesar, Enamine, or Fluorochem and used as received. All NMR spectra were recorded on a Bruker AVANCE III HD 500 One Bay spectrometer with a magnetic field of 11.75 T and a 5 mm SmartProbe BB(F)-H-D. For ¹H NMR spectra, a frequency of 500 MHz resulted. Chemical shifts are reported in ppm from tetramethylsilane as internal standard in CDCl₃ or from [D₆]DMSO as an internal standard (δ = 2.50). Data are reported as follows: chemical shift, multiplicity (s = singlet, d = doublet, t = triplet, q = quartet, quint = quintet, br = broad, m = multiplet), coupling constants (Hz), integration. Purity was assayed by HPLC (Interchim Strategy C-18 column, 4.6mm × 250 mm) with a gradient elution of 5-95% methanol containing 0.2% aqueous acetic acid over 10 minutes with UV detection at λ = 254 nm.

### Example 1:

### General procedures A (amide coupling or sulphonamide)

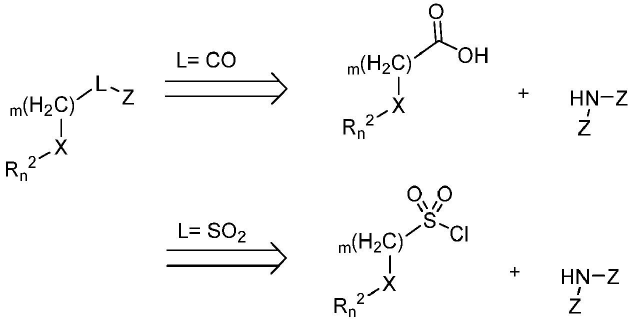

### General synthetic procedure for amide couplings

A solution of carboxylic acid (1 mmol, 1.0 eq), amine (1.1 mmol, 1.1 eq), PyBOP (1.2 mmol, 1.2 eq) in DMF (3 mL) and Et₃N (300 µL) was stirred at ambient temperature for 0.5-16 hours. The reaction mixture was diluted with EtOAc, washed with 0.5 M sat. brine/NaOH (3 × 50 mL), dried (Na₂SO₄), filtered and concentrated *in vacuo.* The residue was purified by either normal phase (SiO₂) or reversed phase (C₁₈) chromatography to isolate the product compound.

### General synthetic procedure for sulphonamides

A solution of sulphonyl chloride (0.2 mmol, 1.0 eq), amine (1.2 eq) in dichloromethane (2.5 mL) and Et3N (80 µL) was stirred at 40 °C overnight. The reaction was diluted with EtOAc and washed with 2 M NaOH aq. (3 × 25 mL), dried (Na2SO4), filtered and concentrated in vacuo. The residue was purified by either normal phase (SiO₂) or reversed phase (C18) chromatography to isolate the title compound.

### Example 2:

### General procedures B (Suzuki coupling)

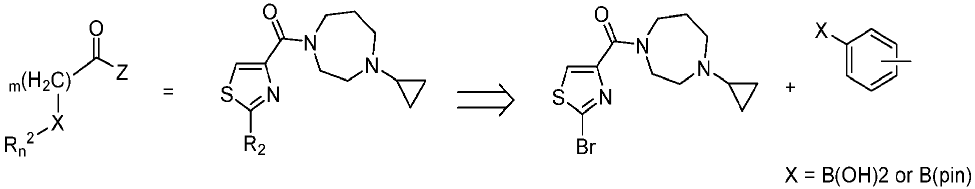

Heteroaryl bromide (0.300 mmol, 1.0 eq) was dissolved in DMF (2 ml) and boronic acid/pinacol boronic ester (1.5 eq), Cs₂CO₃ (4.0 eq) and PdCl₂(PPh₃)₂ (0.10 eq) were added. After the addition of water (1 mL) the mixture was heated at 100 °C between 1 and 16 h. The reaction mixture was concentrated in vacuo and purified purified by either normal phase (SiO₂) or reversed phase (C₁₈) chromatography to isolate the title compound. The was dissolved in MeOH (1 mL) and Si-TMT (0.1 g) added and the suspension was stirred at ambient temperature for 1 h to remove traces of Pd. The suspension was filtered and the filtrate was concentrated in vacuo to dryness.

### Example 3:

### General procedures C (Reductive amination)

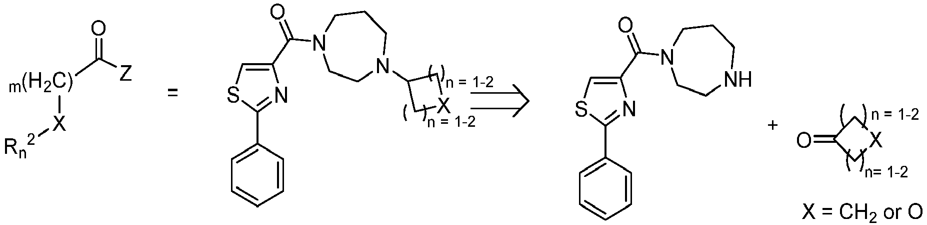

A solution of amine (0.3 mmol, 1.0 eq), ketone (0.33 mmol, 1.1 eq) in 1,2 dichloroethane (5 mL) was stirred at ambient temperature for 30 mins. Sodium triacetoxyborohydride (0.39 mmol, 1.3 eq) was added and stirred at ambient temperature between 1 and 16 h. The reaction mixture was quenched with MeOH (1 mL) and H₂O (1 mL) and subsequently concentrated *in vacuo.* The residue was purified by either normal phase (SiO₂) or reversed phase (C₁₈) chromatography to isolate the title compound.

### Example 4:

### General procedures D (Nucleophilic aromatic substitution)

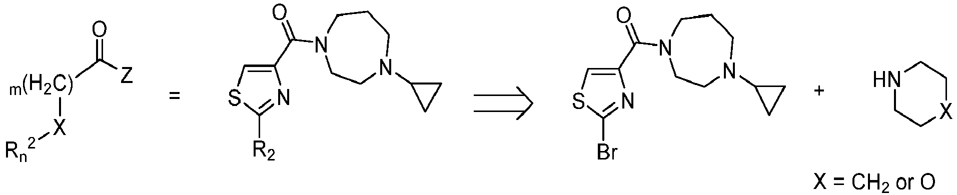

A solution of heteroaryl bromide (0.3 mmol, 1.0 eq) in piperidine (0.5 mL) was held at 80 °C for 4 to 16 h. The reaction mixture was evaporated in vacuo and purified over a silica column to afford the title compound.

### Example 5: Syntheses of compounds

### Synthesis of compound 21560:

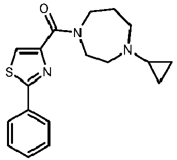

A solution of 2-phenylthiazole-4-carboxylic acid (200 mg, 0.975 mmol, 1.0 eq), 1-cylcopropyl-[1,4]diazepane (150 mg, 1.07 mmol, 1.1 eq), PyBOP (609 mg, 1.17 mmol, 1.2 eq) in DMF (3 mL) and Et₃N (300 µL) was stirred at ambient temperature for 1.5 hours. The reaction mixture was diluted with EtOAc (100 mL), washed with 0.5 M sat. brine/NaOH (3 × 50 mL), dried (Na₂SO₄), filtered and concentrated *in vacuo.* The residue was purified on a SiO₂ column eluting with a gradient of 20 to 70% EtOAc in cyclohexane containing 1% Et₃N to afford a colourless oil (258 mg, 0.788 mmol, 81%).¹H NMR (500 MHz, (CDCl₃): *δ* 7.97-7.92 (m, 2H), 7.90-7.88 (m, 1H), 7.48-7.36 (m, 3H), 3.96-3.92 (m, 1H), 3.91 (t, *J* = 6.2 Hz, 1H), 3.82.3.77 (m, 2H), 3.02-2.98 (m, 2H), 2.02-1.86 (m, 3H), 0.53-0.37 (m, 4H); LRMS (ESI) m/z [M+H]⁺ 328. RP-HPLC: rt 8.08 min purity: 98.0%.

### Synthesis of compound 22815

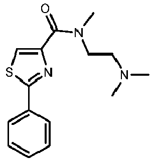

2-phenylthiazole-4-carboxylic acid (50 mg, 0.244 mmol, 1.0 eq) was stirred in SOCl₂ (1 mL) at 40 °C for 30 minutes. Subsequently, thionylchloride was removed *in vacuo.* The residue was dissolved in dichloromethane (2.5 mL) and to this solution *N*,*N*,*N*'-trimethylethylenediamine (34.8 µL, 27.4 mg, 0.268 mmol, 1.1 eq) and Et₃N (100 µL) was added and stirred at ambient temperature for 45 minutes. The reaction mixture was diluted with EtOAc (50 mL), washed with 0.5 M sat. brine/NaOH (3 × 30 mL), dried (Na₂SO₄), filtered and concentrated *in vacuo.* The residue was purified on a SiO₂ column eluting with a gradient of 70 to 100% EtOAc in cyclohexane containing 1% Et₃N to afford a colourless oil (50.0 mg, 0.173 mmol, 71%).¹H NMR (500 MHz, (CDCl₃): Major rotamer: *δ* 7.99-7.90 (m, 3H), 7.48-7.43 (m, 3H), 3.85 (t, *J* = 7.2 Hz, 2H), 3.16 (s, 3H), 2.66 (t, *J* = 7.2 Hz, 2H), 2.20 (s, 6H); minor rotamer: 7.99-7.90 (m, 3H), 7.48-7.43 (m, 3H), 3.69 (t, *J* = 7.0 Hz, 2H), 3.41 (s, 3H), 2.62 (t, *J* = 7.0 Hz, 2H), 2.33 (s, 6H); LRMS (ESI) m/z [M+H]⁺ 290; RP-HPLC: rt 7.53 min purity: 99.3%.

### Synthesis of compound 22745:

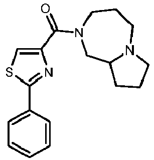

A solution of 2-phenylthiazole-4-carboxylic acid (50 mg, 0.244 mmol, 1.0 eq), octahydro-1*H-*pyrrolo[1,2a][1,4-diazepine] (37.6 mg, 0.268 mmol, 1.1 eq), PyBOP (152 mg, 0.292 mmol, 1.2 eq) in DMF (1 mL) and Et₃N (100 µL) was stirred at ambient temperature for 2 hours. The reaction mixture was diluted with EtOAc (50 mL), washed with 0.5 M sat. brine/NaOH (3 × 30 mL), dried (Na₂SO₄), filtered and concentrated *in vacuo.* The residue was purified on a SiO₂ column eluting with a gradient of 20 to 100% EtOAc (1% Et₃N) in cyclohexane to afford a yellowish oil (75 mg, 0.229 mmol, 94%). ¹H NMR (500 MHz, (CDCl₃): *δ* 7.97-7.92 (m, 2H), 7.92-7.89 (m, 1H), 7.49-7.43 (m, 3H), 4.61-4.55 (m, 0.5H), 4.41-4.34 (m, 0.5H), 4.10-4.00 (m, 1H), 3.91-3.84 (m, 0.5H), 3.73-3.65 (m, 0.5H), 3.24-3.02 (m, 3H), 2.70-2.52 (m, 1H), 2.48-2.32 (m, 2H), 2.15-1.68 (m, 5H), 1.66-1.40 (m, 1H); LRMS (ESI) m/z [M+H]⁺ 328; RP-HPLC: rt 7.74 min purity: 98.2%.

### Synthesis of compound 22747:

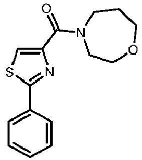

A solution of 2-phenylthiazole-4-carboxylic acid (50 mg, 0.244 mmol, 1.0 eq), 1,4-oxetane (27.1 mg, 0.268 mmol, 1.1 eq), PyBOP (152 mg, 0.292 mmol, 1.2 eq) in DMF (1 mL) and Et₃N (100 µL) was stirred at ambient temperature for 3 hours. The reaction mixture was diluted with EtOAc (50 mL), washed with 0.5 M sat. brine/NaOH (3 × 30 mL), dried (Na₂SO₄), filtered and concentrated *in vacuo.* The residue was purified on a SiO₂ column eluting with a gradient of 20 to 70% EtOAc in cyclohexane to afford a colourless oil (68 mg, 0.236 mmol, 97%). ¹H NMR (500 MHz, (CDCl₃): *δ* 7.98-7.90 (m, 3H), 7.49-7.44 (m, 3H), 4.06-4.00 (m, 2H), 3.98-3.94 (m, 1H), 3.90-3.83 (m, 5H), 2.14-2.05 (m, 2H), LRMS (ESI) m/z [M+H]⁺ 289; RP-HPLC: rt 12.47 min purity: 98.7%.

### Synthesis of compound 22817:

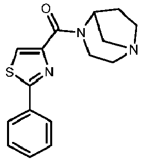

2-phenylthiazole-4-carboxylic acid (50 mg, 0.244 mmol, 1.0 eq) was stirred in SOCl₂ (1 mL) at 40 °C for 30 minutes. Subsequently, thionylchloride was removed *in vacuo.* The residue was dissolved in dichloromethane (2.5 mL) and to this solution 1,4-diazabicyclo[3.2.1]octane dihydrochlorid (49.6 mg, 0.268 mmol, 1.1 eq) and Et₃N (200 µL) was added and stirred at ambient temperature for 45 minutes. The reaction mixture was diluted with EtOAc (50 mL), washed with 0.5 M sat. brine/NaOH (3 × 30 mL), dried (Na₂SO₄), filtered and concentrated *in vacuo.* The residue was purified on a SiO₂ column eluting with a gradient of 80 to 100% EtOAc in cyclohexane containing 1% Et₃N to afford a colourless solid (45.2 mg, 0.151 mmol, 62%). ¹H NMR (500 MHz, (CDCl₃): *δ* 8.02 (s, 1H), 7.96-7.87 (m, 32), 7.50-7.43 (m, 3H), 5.69-5.47 (m 1H), 4.98-4.45 (m, 1H), 4.35-4.27 (m, 1H), 3.58-3.07 (m, 6H), 2.46-2.14 (m, 3H). LRMS (ESI) m/z [M+H]⁺ 300; RP-HPLC: rt 7.31 min purity: 99.1%.

### Synthesis of compound 22393:

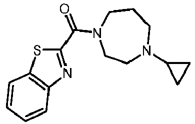

A solution of potassium 1,3-benzothiazole-2-carboxylate (50 mg, 0.230 mmol, 1.0 eq), 1cylcopropyl-[1,4]diazepane (35.5 mg, 0.253 mmol, 1.1 eq), PyBOP (144 mg, 0.276 mmol, 1.2 eq) in DMF (1 mL) and Et₃N (100 µL) was stirred at ambient temperature for 16 hours. The reaction mixture was diluted MeOH (1 mL) and H₂O (1 mL) and directly purified on a C₁₈ column eluting with a gradient of 10 to 100% AcN in H₂O afforded a colourless oil, which still was impure. A second purification on a SiO₂ column eluting with a gradient of 50 to 100% EtOAc (1% Et₃N) in cyclohexane afford a yellowish oil (46 mg, 0.153 mmol, 66%). ¹H NMR (500 MHz, (CDCl₃): *δ* 8.11-8.06 (m, 1H), 7.98-7.94 (m, 1H), 7.55-7.51 (m, 1H), 7.50-7.45 (m, 1H), 4.33-4.25 (m, 2H), 3.86-3.80 (m, 2H), 3.04-2.98 (m, 2H), 2.89-2.83 (m, 2H), 2.04-1.83 (m, 3H), 0.53-0.37 (m, 4H). LRMS (ESI) m/z [M+H]⁺ 302. RP-HPLC: rt 7.88 min purity: 99.8%.

### Synthesis of compound 22396:

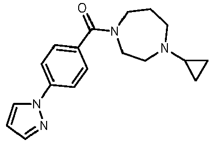

A suspension of 4-(1*H*-pyrazol-1-yl)benzoic acid (50 mg, 0.266 mmol, 1.0 eq), 1-cylcopropyl-[1,4]diazepane (41.0 mg, 0.292 mmol, 1.1 eq), PyBOP (166 mg, 0.319 mmol, 1.2 eq) in DMF (1 mL) and Et₃N (100 µL) was stirred at ambient temperature for 1 hour. The reaction mixture was diluted with EtOAc (50 mL), washed with 0.5 M sat. brine/NaOH (3 × 30 mL), dried (Na₂SO₄), filtered and concentrated *in vacuo.* The residue was purified on a SiO₂ column eluting with a gradient of 50 to 100% EtOAc (1% Et₃N) in cyclohexane afford a colourless oil (69 mg, 0.193 mmol, 73%). ¹H NMR (500 MHz, (CDCl₃): *δ* 7.97-7.94 (m, 1H), 7.76-7.72 (m, 3H), 7.53-7.48 (m, 2H), 6.49 (dd, *J* = 2.4, 1.8 Hz), 3.80-3.75 (m, 2H), 3.51-3.44 (m, 2H), 3.01-2.95 (m, 1H), 2.90-2.85 (m, 1H), 2.83-2.76 (m, 3H), 1.99-1.73 (m, 3H), 0.53-0.34 (m, 4H). LRMS (ESI) m/z [M+H]⁺ 311. RP-HPLC: rt 6.42 min purity: 99.7%.

### Synthesis of compound 22608:

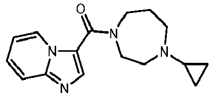

A solution of imidazo[1,2-a]pyridine-3-carboxylic acid (50 mg, 0.308 mmol, 1.0 eq), 1-cylcopropyl-[1,4]diazepane (47.6 mg, 0.339 mmol, 1.1 eq), PyBOP (193 mg, 0.370 mmol, 1.2 eq) in DMF (1.5 mL) and Et₃N (100 µL) was stirred at ambient temperature over the weekend. The reaction mixture was diluted with EtOAc (50 mL), washed with 0.5 M sat. brine/NaOH (3 × 30 mL), dried (Na₂SO₄), filtered and concentrated *in vacuo.* The residue was purified on a SiO₂ column eluting with a gradient of 30 to 100% EtOAc (1% Et₃N) in cyclohexane to afford a colourless oil (47 mg, 0.144 mmol, 47%). ¹H NMR (500 MHz, (CDCl₃): *δ* 9.15 (d, *J* = 7.1 Hz, 1H), 7.89 (s, 1H), 7.68 (dt, *J* = 9.0, 1.1 Hz, 1H), 7.34 (ddd, *J* = 7.9, 6.9, 1.3 Hz, 1H), 6.94 (td, *J* = 6.9, 1.2 Hz, 1H), 3.97-3.76 (m, 4H), 3.04-2.96 (m, 2H), 2.89-2.80 (m, 2H), 2.01-1.94 (m, 2H), 1.90-1.84 (m, 1H), 0.54-0.38 (m, 4H). LRMS (ESI) m/z [M+H]⁺ 285; RP-HPLC: rt 4.75 min purity: 93.6%.

### Synthesis of compound 22635:

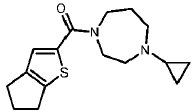

A solution of 5,6-dihydro-4*H*-cyclopenta[b]thiophene-2-carboxylic acid (50 mg, 0.297 mmol, 1.0 eq), 1-cylcopropyl-[1,4]diazepane (45.9 mg, 0.373 mmol, 1.1 eq), PyBOP (186 mg, 0.357 mmol, 1.2 eq) in DMF (2.5 mL) and Et₃N (100 µL) was stirred at ambient temperature for 2 hours. The reaction mixture was diluted with EtOAc (50 mL), washed with 0.5 M sat. brine/NaOH (3 × 30 mL), dried (Na₂SO₄), filtered and concentrated *in vacuo.* The residue was purified on a SiO₂ column eluting with a gradient of 30 to 100% EtOAc (1% Et₃N) in cyclohexane to afford a colourless oil (37 mg, 0.127 mmol, 43%). ¹H NMR (500 MHz, (CDCl₃): *δ* 7.06 (s, 1H), 3.86-3.68 (m, 4H), 2.95-2.88 (m, 4H), 2.84-2.80 (m, 2H), 2.76-2.71 (m, 2H), 2.48-2.41 (m, 2H), 1.95-1.89 (m, 2H), 1.88-1.82 (m, 1H), 0.51-0.37 (m, 4H); LRMS (ESI) m/z [M+H]⁺ 291; RP-HPLC: rt 7.56 min purity: 99.5%.

### Synthesis of compound 22741:

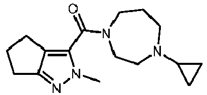

A solution of 2-Methyl-2,4,5,6-tetrahydro-cyclopentapyrazole-3-carboxylic acid (53 mg, 0.319 mmol, 1.0 eq), 1-cylcopropyl-[1,4]diazepane (49.3 mg, 0.352 mmol, 1.1 eq), PyBOP (200 mg, 0.384 mmol, 1.2 eq) in DMF (1 mL) and Et₃N (100 µL) was stirred at ambient temperature for 1 hour. The reaction mixture was diluted with EtOAc (50 mL), washed with 0.5 M sat. brine/NaOH (3 × 30 mL), dried (Na₂SO₄), filtered and concentrated *in vacuo.* The residue was purified on a SiO₂ column eluting with a gradient of 10 to 70% EtOAc (1% Et₃N) in cyclohexane to afford colourless oil (43 mg, 0.149 mmol, 47%).¹H NMR (500 MHz, (CDCl₃): *δ* 3.89-3.85 (m, 3H), 3.75-3.69 (m, 2H), 3.59-3.53 (s, 2H), 2.96-2.92 (m, 1H), 2.86-2.81 (m, 1H), 2.81-2.73 (m, 2H), 2.73-2.68 (m, 2H), 2.65-2.58 (m, 2H), 2.44-2.36 (m, 2H), 1.96-1.89 (m, 1H), 1.87-1.77 (m, 2H), 0.52-0.35 (m, 4H); LRMS (ESI) m/z [M+H]⁺ 289; RP-HPLC: rt 6.18 min purity: 98.7%.

### Synthesis of compound 22742

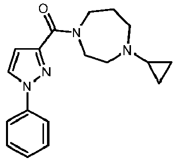

A solution of 1-phenyl-1*H*-pyrazole-3-carboxylic acid (50 mg, 0.266 mmol, 1.0 eq), 1-cylcopropyl-[1,4]diazepane (41.0 mg, 0.292 mmol, 1.1 eq), PyBOP (166 mg, 0.319 mmol, 1.2 eq) in DMF (1 mL) and Et₃N (100 µL) was stirred at ambient temperature for 1 hour. The reaction mixture was diluted with EtOAc (50 mL), washed with 0.5 M sat. brine/NaOH (3 × 30 mL), dried (Na₂SO₄), filtered and concentrated *in vacuo.* The residue was purified on a SiO₂ column eluting with a gradient of 10 to 60% EtOAc (1% Et₃N) in cyclohexane to afford a colourless oil (67 mg, 0.216 mmol, 81%).¹H NMR (500 MHz, (CDCl₃): *δ* 7.92 (d, *J* = 2.6 Hz), 7.71-7.67 (m, 2H), 7.50-7.45 (m, 2H), 7.35-7.30 (m, 1H), 6.92-6.89 (m, 1H), 4.07-3.99 (m, 2H), 3.82-3.77 (m, 2H), 3.03-2.97 (m, 2H), 2.89-2.84 (m, 2H), 2.81-2.73 (m, 2H), 2.04-1.94 (m, 2H), 1.94-1.85 (m, 1H), 0.51-0.39 (m, 4H); LRMS (ESI) m/z [M+H]⁺ 311; RP-HPLC: rt 7.48 min purity: 96.7%.

### Synthesis of compound 22749:

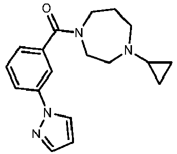

A solution of 3-(1*H*-pyrazol-1-yl)benzoic acid (50 mg, 0.266 mmol, 1.0 eq), 1-cylcopropyl-[1,4]diazepane (41.0 mg, 0.292 mmol, 1.1 eq), PyBOP (166 mg, 0.319 mmol, 1.2 eq) in DMF (1 mL) and Et₃N (100 µL) was stirred at ambient temperature for 1.5 hours. The reaction mixture was diluted with EtOAc (50 mL), washed with 0.5 M sat. brine/NaOH (3 × 30 mL), dried (Na₂SO₄), filtered and concentrated *in vacuo.* The residue was purified on a SiO₂ column eluting with a gradient of 30 to 100% EtOAc (1% Et₃N) in cyclohexane to afford a colourless oil (44 mg, 0.178 mmol, 56%).¹H NMR (500 MHz, (CDCl₃): *δ* 7.96-7.93 (m, 1H), 7.78-7.71 (m, 3H), 7.51-7.46 (m, 1H), 7.31-7.28 (m, 1H), 6.48 (dd, *J* = 2.4, 1.8 Hz, 1H), 3.81-3.75 (m, 2H), 3.51-3.45 (m, 2H), 3.11-3.05 (m, 1H), 3.01-2.95 (m, 1H), 2.90-2.84 (m, 1H), 2.84-2.76 (m, 2H), 1.99-1.75(m, 3H), 0.54-0.35 (m, 4H); LRMS (ESI) m/z [M+H]⁺ 310; RP-HPLC: rt 6.51 min purity: 95.6%.

### Synthesis of compound 22394:

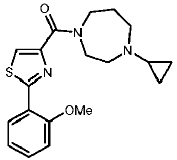

A solution of 2-(pyridin-2-yl)-1,3-thiazole-4-carboxylic acid (50 mg, 0.242 mmol, 1.0 eq), 1-cylcopropyl-[1,4]diazepane (37.4 mg, 0.291 mmol, 1.1 eq), PyBOP (151.4 mg, 0.291 mmol, 1.2 eq) in DMF (1 mL) and Et₃N (100 µL) was stirred at ambient temperature for 1 hour. The reaction mixture was diluted MeOH (1 mL) and H₂O (1 mL) and directly purified on a C₁₈ column eluting with a gradient of 10 to 100% AcN in H₂O afforded a colourless oil, which still was impure. A second purification on a SiO₂ column eluting with a gradient of 50 to 100% EtOAc (1% Et₃N) in cyclohexane afford a colourless oil (53 mg, 0.161 mmol, 67%). ¹H NMR (500 MHz, (CDCl₃): *δ* 8.35 (dd, *J* = 7.9, 1.6 Hz, 1H), 7.97-7.94 (m, 1H), 7.45-7.38 (m, 1H), 7.13-7.02 (m, 2H), 4.05 (s, 3H), 3.98-3.89 (m, 2H), 3.82-3.77 (m, 2H), 3.04-2.98 (m, 2H), 2.91-2.85 (m, 2H), 2.03-1.86 (m, 3H), 0.52-0.38 (m, 4H). LRMS (ESI) m/z [M+H]⁺ 358. RP-HPLC: rt 8.42 min purity: 99.3%.

### Synthesis of compound 22612:

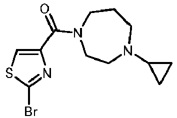

To an ice-cold suspension of 2-bromo-4-thiazolecarboxylic acid (3.27 g, 15.7 mmol, 1.1 eq) in dichloromethane (20 mL) was dropwise added oxalyl chloride (1.4 mL, 16.3 mmol, 1.1 eq) followed by addition of DMF (0.01 mL, 0.129 mmol, 0.01 eq) (caution: vigorous gas evolution). The mixture was stirred for 2 h at ambient temperature and susbsequently cooled to 0 °C. A solution of 1-cyclopropyl-1,4-diazepane (1.98 mg, 14.1 mmol, 1.0 eq) and Et₃N (5 mL, 35.9 mmol, 2.54 eq) in dichloromethane (20 mL) was added to the ice-cold suspension. The resulting solution was allowed to warm to ambient temperature and stirred for 1h. The reaction mixture was cooled again to 0 °C and quenched with 1/1 Brine/1 M NaOH (30 mL). The layers were separated, the aq. layer was washed with dichloromethane (3 × 70 mL). The combined org. layers were dried (Na₂SO₄), filtered and concentrated in *vacuo.* The residue was purified by silica column eluting with 20 to 80 % EtOAc in cyclohexane containing 1% TEA to afford a yellow liquid (3.11 g, 9.43 mmol, 66%). ¹H-NMR (500 MHz, CDCl₃): *δ* 7.87-7.80 (m, 1H), 3.84-3.70 (m, 2H),2.97-2.90 (m, 2H), 2.86-2.79 (m, 2H), 1.97-1.82 (m, 3H), 0.51-0.37 (m, 4H). LRMS (ESI) m/z [M+H]⁺ 330. RP-HPLC: rt 5.91 min purity: 96.7%.

### Synthesis of compound 22850:

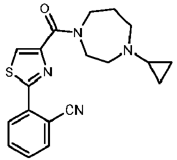

1-(2-bromo-1,3-thiazole-4-carbonyl)-4-cyclopropyl-1,4-diazepane (101 mg, 0.306 mmol, 1.0 eq) was dissolved in DMF (2 ml) and 2-cyanobenzenboronic acid (69 mg, 0.470 mmol, 1.5 eq), Cs₂CO₃ (405 mg, 1.24 mmol, 4.1 eq) and PdCl₂(PPh₃)₂ (21 mg, 0.030 mmol, 0.10 eq) are added. After the addition of water (1 ml) the remaining mixture was heated to 100° C. LC-MS after 1 h showed complete conversion. The reaction mixture was evaporated with Isolute HM-N. The residue was purified by silica column (24 g) eluting with 0 to 5% MeOH in DCM with 1% TEA. The product was dissolved again in MeOH (1 mL), Si-TMT resin (0.1 g) was added and the suspension was stirred at RT for 1 h to remove Pd traces. The suspension was filtered and the filtrate was concentrated in vacuo to dryness to give a greenish oil, which became an amorphous green solid (48 mg, 0.136 mmol, 45%). ¹H-NMR (500 MHz, CDCl₃): *δ* 7.99-7.98 (m, 1H), 7.73-7.69 (m, 1H), 7.69-7.66 (m, 1H), 7.57-7.50 (m, 2H), 3.94-3.87 (m, 2H), 3.80-75 (m, 2H), 2.99-2.95 (m, 1H), 2.94-2.90 (m, 1H), 2.87-79 (m, 2H), 1.99-1.81 (m, 3H), 0.51-0.37 (m, 4H). LRMS (ESI) m/z [M+H₂O+H]⁺ 371. RP-HPLC: rt 5.96 min purity: 96.0%.

### Materials and Methods

### Mouse MLL-AF9 compound screening

For testing the small molecule compounds targeting CD93 on GMPs (leukemia stem cells) colony forming capacity, 10³ FACS-purified GFP+lin-Sca-1-c-kithighCD34+Fcγ+ GMPs from BL6 or CD93-/- transduced AML mice were cultured overnight in 96-well U-bottom plates (NeoLab) in RPMI medium supplemented with 10% FCS, 1% L-glutamine, 1% penicillin/streptomycin, 100 ng/ml recombinant mouse (rm)-SCF (Miltenyi) and 20 ng/ml rm-TPO (ProSpec), in the presence or absence of 10 uM and 1 uM compound in technical triplicates. Compounds were dissolved in DMSO. The next day, cells were plated in semi-solid methylcellulose in the absence of any compound. Number of colonies was assessed by inverted light microscopy after 7 days.

In this assay, the semi-solid Media used was 1.27% Methylcellulose (1500cp, Sigma), supplemented with 15% FCS, 20% BIT (50 mg/ml BSA in IMDM, 1.44 U/ml rh-insulin [Actrapid; Novo Nordisk], and 250 ng/ml human holo transferrin [Prospec]), 100 µM 2-mercaptoethanol, 100 U/ml penicillin, 100 µg/ml streptomycin, 2 mM L-glutamine, and 50 ng/ml rm-SCF (Miltneyi), 10 ng/ml rm-IL-3 (Miltenyi), 10 ng/ml rm-IL-6 (Miltenyi), and 50 ng/ml rm-Flt3-ligand (ProSpec).

As shown beforehand for the CD93 inhibitor metoclopramide, treatment of leukemia stem cells derived from a murine model of chronic myeloid leukemia using BL6 and CD93^{-/-} mice reduced colony formation of BL6 leukemia stem cells, and concomitantly the treatment of human CML with metoclopramide diminished leukemia stem cell numbers and function (6).

### Compound screening on human AML and HD cells

5×10³ FACS-purified CD45dimSSCIolin-CD34+ AML stem and progenitor cells from BM and PB of AML patients were cultured overnight in 96-well U-bottom plates (NeoLab) in Stem Span Medium (STEMCELL Technologies) supplemented with 100X Stem Span Cytokine Cocktail (STEMCELL Technologies), in the presence or absence of 50 uM, 10 uM and 2 uM compound in technical triplicates. Compounds were dissolved in DMSO. The next day, cells were plated in semi-solid methylcellulose in the absence of any compound. Number of colonies was assessed by inverted light microscopy after 14 days.

In this assay, the semi-solid Media used was Human Methylcellulose Enriched Media Complete with G-CSF and IL-6 for the differentiation and enumeration of CD34+ human hematopoietic stem cell (R&D Systems).

### Cited prior art documents:

All scientific publications and patent documents cited in the present specification are incorporated by reference herein.
1. Holyoake TL, Vetrie D. The chronic myeloid leukemia stem cell: stemming the tide of persistence. Blood. 2017 Mar 23;129(12):1595-1606. doi: 10.1182/blood-2016-09-696013. Epub 2017 Feb 3. PMID: 28159740.
2. Kinstrie, R., Horne, G.A., Morrison, H. et al. CD93 is expressed on chronic myeloid leukemia stem cells and identifies a quiescent population which persists after tyrosine kinase inhibitor therapy. Leukemia 34, 1613-1625 (2020). https://doi.org/10.1038/s41375-019-0684-5.
3. Sambrook et al., Molecular Cloning: A Laboratory Manual, 4th ed. (2012) Cold Spring Harbor Laboratory Press, Cold Spring Harbor, N.Y.
4. Ausubel et al., Short Protocols in Molecular Biology (2002) 5th Ed, John Wiley & Sons, Inc.).
5. L. Lachman et al. The Theory and Practice of Industrial Pharmacy, 4th Ed, 2013 (ISBN 8123922892).
6. Riether, Carsten, et al. "Metoclopramide treatment blocks CD93-signaling-mediated self-renewal of chronic myeloid leukemia stem cells." Cell reports 34.4 (2021): 108663.

## Claims

1. A compound of formula (A) 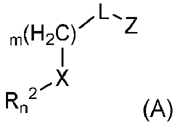 wherein
L is selected from -CO, -SO₂ or -(CH₂)_{q},
Z is selected from any one of the moieties 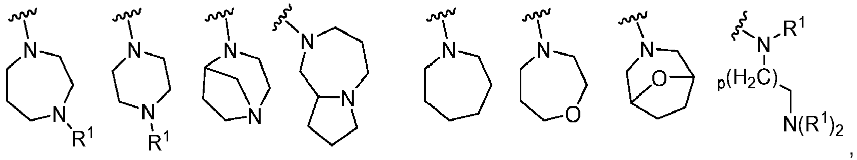
R¹ is selected from
- -C₃-C₆ cycloalkyl,
- H,
- C₁-C₆- alkyl,
- haloalkanes, particularly fluorinated alkanes,
- a heterocycle, wherein the heterocycle comprises 5 or 6 atoms and up to 2 heteroatoms, and
- -COCH₃,
X is selected from
- a heteroaryl,
- phenyl, and
- SP-phenyl or SP-heteroaryl, wherein SP is an alkyne or alkene,
Rₙ² is selected from
- -C₁-C₄ alkyl, particularly methyl,
- an aromatic moiety (Ar), wherein Ar is an substituted or unsubstituted aryl or substituted or unsubstituted heteroaryl,
- the group of halogens, particularly Br,
- a heterocycle, wherein the heterocycle comprises 5 or 6 atoms and up to 3 heteroatoms, and
- -NMe₂, -OMe, -SO₂Me, -CONHMe, -NH-Phenyl,
m is 0 or 1, particularly 0,
n is 0, 1 or 2, particularly 0 or 1,
p is 1 or 2,
q is 0, 1 or 2, particularly 0 or 1.

2. The according to claim 1, wherein the compound is a compound of formula (I), (II), or (III) , 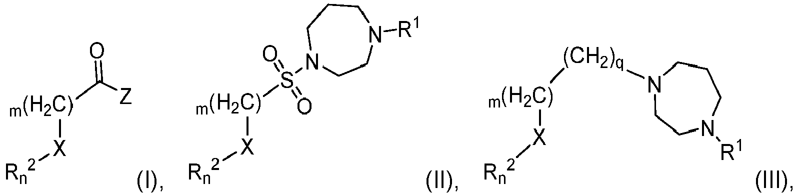 wherein
Z is selected from 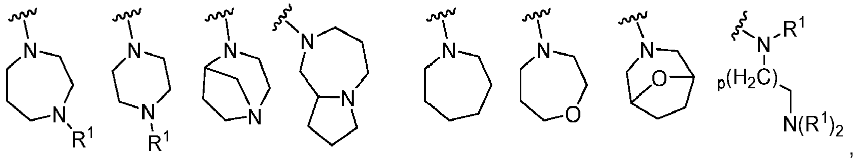
R¹ is selected from
- -C₃-C₆ cycloalkyl,
- -H,
- -C₁-C₆- alkyl,
- haloalkanes, particularly fluorinated alkanes,
- a heterocycle, wherein the heterocycle comprises 5 or 6 atoms and up to 3 heteroatoms, and
- -COCH₃,
X is selected from
- a heteroaryl,
- phenyl, and
- SP-phenyl or SP-heteroaryl, wherein SP is an alkyne or alkene,
Rₙ² is selected from
- -C₁-C₄ alkyl, particularly methyl,
- an aromatic moiety (Ar), wherein Ar is an substituted or unsubstituted aryl or substituted or unsubstituted heteroaryl
- the group of halogens, particularly Br,
- a heterocycle, wherein the heterocycle comprises 5 or 6 atoms and up to 3 heteroatoms, particularly up to 2 heteroatoms, and
- -NMe₂, -OMe, -SO₂Me, -CONHMe, -NH-Phenyl,
m is 0 or 1, particularly 0,
n is 0, 1 or 2, particularly 0 or 1,
p is 1 or 2,
q is 0, 1 or 2, particularly 0 or 1.

3. The compound according to any of the preceding claims, wherein R¹ is selected from
- -C₃-C₆ cycloalkyl, particularly cyclopropyl,
- -H, and
- -C₁-C₆- alkyl,
particularly from
- -C₃-C₆ cycloalkyl, particularly cyclopropyl, and
- -C₁-C₆- alkyl,
more particularly from
- -C₃-C₆ cycloalkyl, particularly cyclopropyl.

4. The compound according to any of the preceding claims, wherein X is a phenyl or heteroaryl and wherein the heteroaryl comprises 5 to 10 atoms.

5. The compound for use in the treatment of cancer according to any of the preceding claims wherein the heteroaryl of R² and the heteroaryl of X independently comprise up to 3 heteroatoms, particularly up to 2 heteroatoms, more particularly 2 heteroatoms.

6. The compound according to any of the preceding claims, wherein X-Rₙ² is selected from any one of the moieties 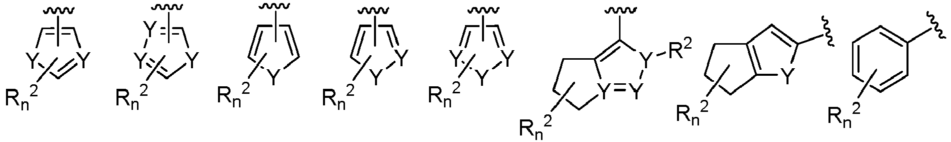 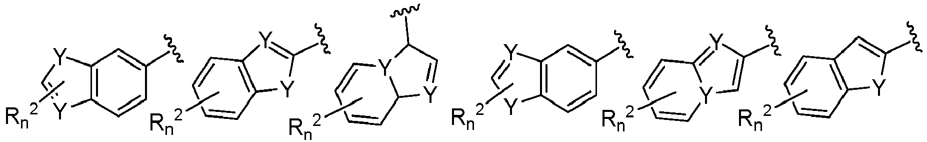 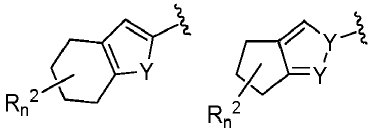 wherein
Y is N, O or S,
R² is defined as above,
n is 0, 1 or 2, particularly 0 or 1,
in particular -Rₙ² is selected from any one of the moieties 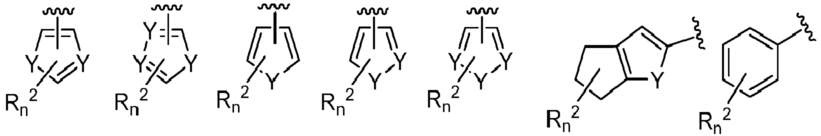 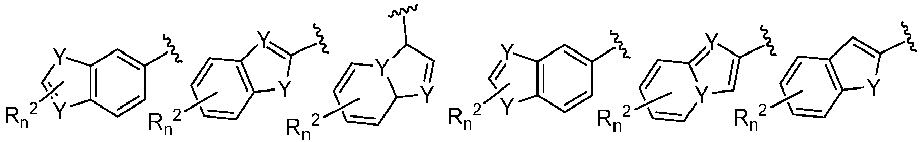 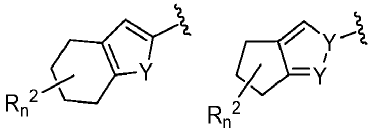 wherein
Y is N, O or S,
R² is defined as above,
n is 0, 1 or 2, particularly 0 or 1.

7. The compound according to any of the preceding claims, wherein X-Rₙ² is selected from any one of the moieties 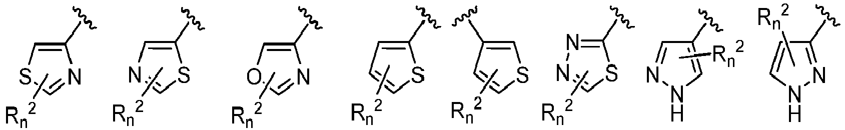 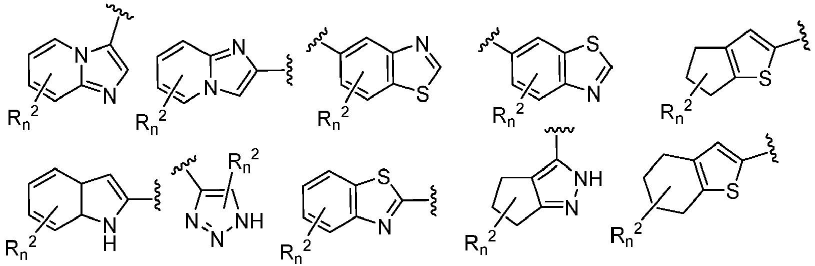 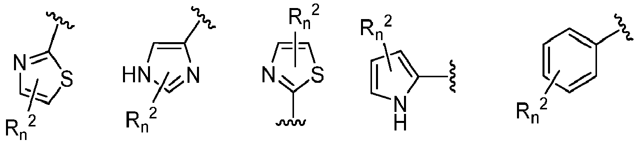 wherein
R² is defined as above,
n is 0 or 1.

8. The compound according to any of the preceding claims, wherein X-R² is selected from any one of the moieties 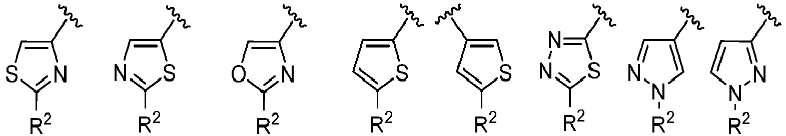 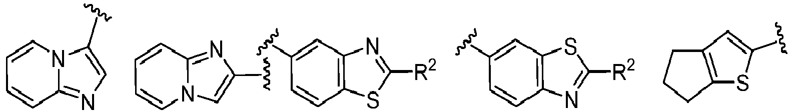 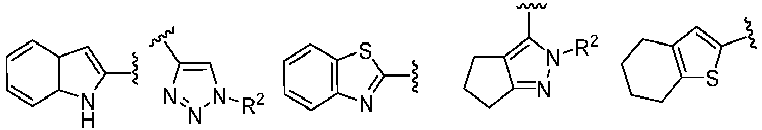 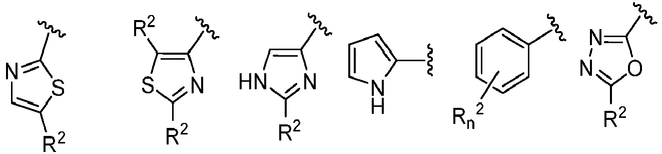 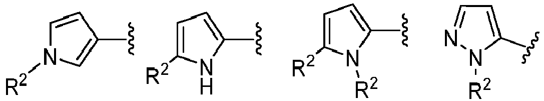 particularly from any one of the moieties 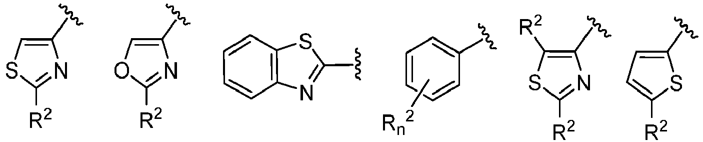 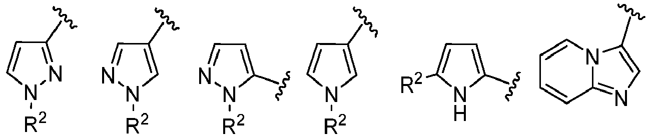 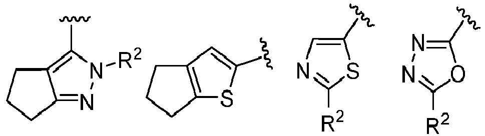 wherein
R² is defined as above,
n is 0 or 1.

9. The compound according to any of the preceding claims, wherein R² is selected from
- C₁-C₄ alkyl, particularly methyl,
- Ar, wherein Ar is a substituted or unsubstituted aryl or a substituted or unsubstituted heteroaryl
- the group of halogens, particularly Br
- a heterocycle, wherein the heterocycle comprises 5 or 6 atoms and up to 2 heteroatoms, particularly piperidine, and
- -NMe₂, -OMe, -SO₂Me, -CONHMe, -NH-Phenyl.
particularly from
- C₁-C₄ alkyl, particularly methyl,
- Ar, wherein Ar is a substituted or unsubstituted aryl or a substituted or unsubstituted heteroaryl
- a heterocycle, wherein the heterocycle comprises 5 or 6 atoms and up to 2 heteroatoms, particularly piperidine, and
- the group of halogens, particularly Br.

10. The compound according to any of the preceding claims, wherein the substituted aryl or the substituted heteroaryl is substituted with Rₛ³, wherein s is 0, 1 or 2, particularly 0 or 1.

11. The compound according to any of the preceding claims, wherein R³ is selected from
- C₁-C₄ alkyl, particularly methyl,
- the group of halogens, particularly F or Cl, and
- -CN, -NO₂, -OCF₃, -NMe₂, -CONHMe, -OMe, -SO₂Me, -NHSO₂Me, - CF₃, -Me.

12. The compound according to any of the preceding claims, wherein the compound is a compound of formula (IV), (IVa), (V), or (VII), particularly of (IV), (V), or (VII) 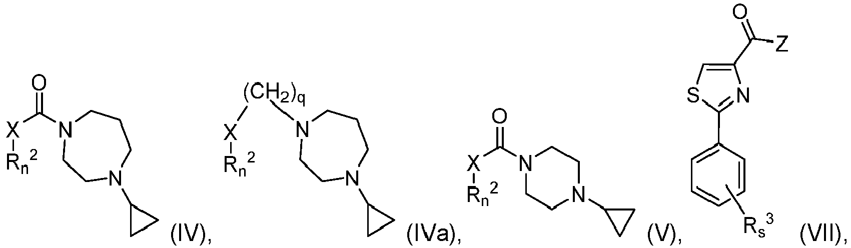 Wherein
q is 0 or 1, particularly 0,
X is selected from any one of the moieties 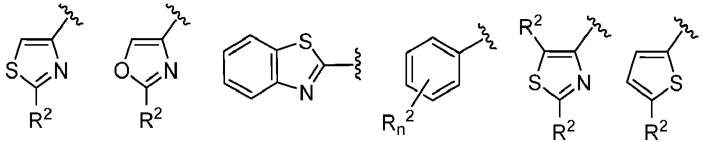 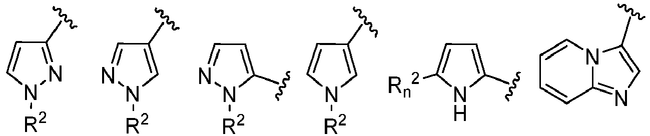 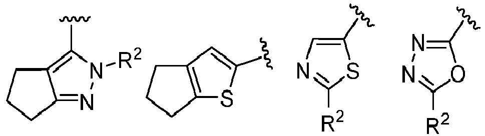
R² is selected from
- -C₁-C₄ alkyl, particularly methyl,
- Ar, wherein Ar is a substituted or unsubstituted aryl or a substituted or unsubstituted heteroaryl,
- a heterocycle, wherein the heterocycle comprises 5 or 6 atoms and up to 2 heteroatoms, particularly piperidine, and
- the group of halogens, particularly Br,
R³ is selected from
- -C₁-C₄ alkyl, particularly methyl,
- the group of halogens, particularly F or Cl, and
- -CN, -NO₂, -OCF₃, -NMe₂, -CONHMe, -OMe, -SO₂Me, -NHSO₂Me, -CF₃, -Me,
Z is selected from any one of the moieties 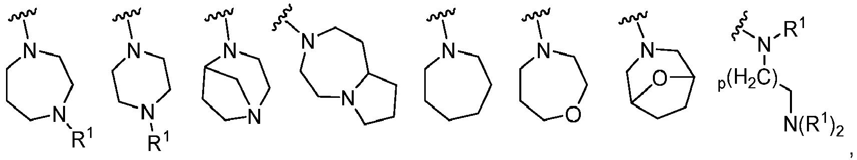
R¹ is selected from
- methyl and cyclopropyl,
n is 0 or 1,
p is 1 or 2,
s is 0 or 1.

13. The compound according to any of the preceding claims, wherein the compound is a compound of formula (IV), (IVa), (V) or (VII), particularly of formula (IV),or (VIII) 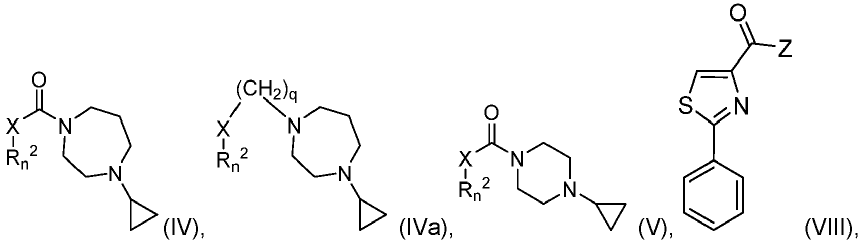 wherein
q is 0 or 1, particularly 0,
X is selected from any one of the moities 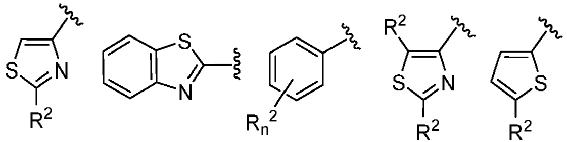 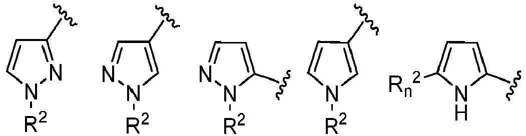 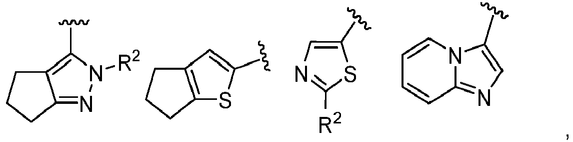
particularly from any one of the moieties 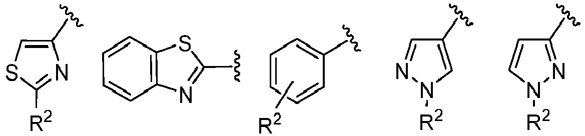 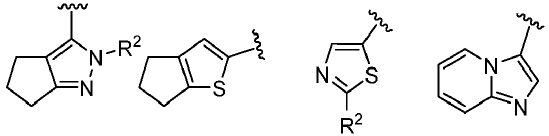
R² is selected from
- -C₁-C₄ alkyl, particularly methyl,
- the group of halogens, particularly Br, and
- any one of the moieties 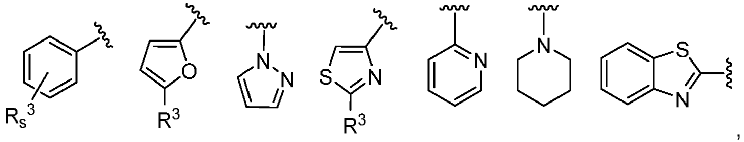 particularly from
- methyl, Br, and
- 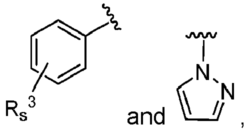
R³ is selected from
- methyl, -OMe, -NHSO₂Me, -Cl, -NO₂, and -CN,
particularly from
- -OMe, and -CN,
Z is selected from any one of the moieties 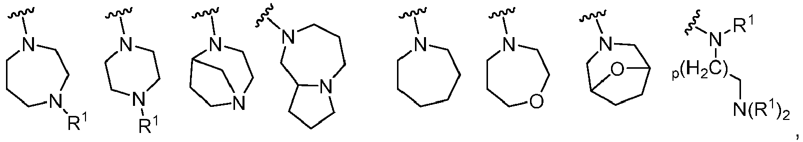 particularly from any one of the moieties 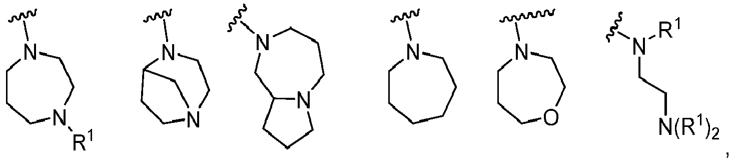 more particularly from any one of the moieties 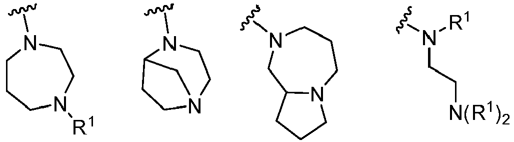
R¹ is selected from
- methyl and cyclopropyl,
n is 0 or 1, particularly 1,
p is 1 or 2,
s is 0 or 1.

14. A compound according to claims 1 to 13, for use as a medicament.

15. A compound according to claims 1 to 13, for use in the treatment of a disease, wherein the disease is cancer, more particularly leukemia, lymphoma or myeloma.
